(19) Europäisches Patentamt
European Patent Office
Office européen des brevets

(11) Veröffentlichungsnummer: **0 036 555**
**A1**

(12) # EUROPÄISCHE PATENTANMELDUNG

(21) Anmeldenummer: **81101745.8**

(22) Anmeldetag: **10.03.81**

(51) Int. Cl.³: **C 07 C 119/00**, C 07 D 265/38, C 09 B 19/00

(30) Priorität: **22.03.80 DE 3011154**

(43) Veröffentlichungstag der Anmeldung: **30.09.81 Patentblatt 81/39**

(84) Benannte Vertragsstaaten: **BE CH DE FR GB IT LI**

(71) Anmelder: **HOECHST AKTIENGESELLSCHAFT, Postfach 80 03 20, D-6230 Frankfurt/Main 80 (DE)**

(72) Erfinder: **Mohr, Reinhard, Dr., Rohrstrasse 34, D-6050 Offenbach/Main (DE)**
Erfinder: **Neeb, Rudolf, Dr., Alexanderstrasse 26, D-6053 Obertshausen (DE)**

(54) **Neue Zinkchloridkomplexverbindungen, Verfahren zu ihrer Herstellung sowie ihre Verwendung und Verfahren zur Herstellung von Phenoxazinverbindungen.**

(57) Zinkchloridkomplexverbindungen, denen die Struktur der allgemeinen Formel (1)

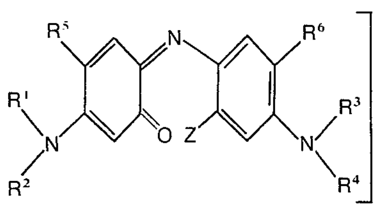

· $ZnCl_2$ (1)

zugeordnet werden kann, wobei die einzelnen Formelreste bedeuten:

$R^1$ ist ein Wasserstoffatom oder eine gegebenenfalls substituierte niedere Alkylgruppe;

$R^2$ ist ein Wasserstoffatom, eine gegebenenfalls substituierte niedere Alkylgruppe oder ein gegebenenfalls substituierter Phenylrest;

$R^3$ ist ein Wasserstoffatom oder eine gegebenenfalls niedere Alkylgruppe;

$R^4$ ist ein Wasserstoffatom, eine gegebenenfalls substituierte niedere Alkylgruppe oder ein gegebenenfalls substituierter Phenylrest;

$R^5$ ist ein Wasserstoff- oder ein Halogenatom oder eine niedere Alkyl- oder eine niedere Alkoxygruppe, oder $R^1$ und $R^5$ bilden zusammen den o-Phenylenrest;

$R^6$ ist ein Wasserstoff- oder ein Halogenatom, oder eine niedere Alkyl- oder eine niedere Alkoxygruppe;

Z ist ein Wasserstoff- oder ein Halogenatom oder eine niedere Alkylgruppe oder eine Cyan-, Carbonsäure-, Carbamoyl-, Sulfamoyl-, eine durch niederes Alkyl mono- oder disubstituierte Carbamoyl- oder Sulfamoylgruppe oder eine Trifluormethyl- oder niedere Carbalkoxygruppe, eine niedere Alkoxygruppe, die durch Cyan substituiert sein kann, eine Acyloxygruppe, eine Acylaminogruppe, eine niedere Alkylaminogruppe oder eine niedere Dialkylaminogruppe. Sie werden hergestellt, indem man eine Nitrosoverbindung der Formel (2)

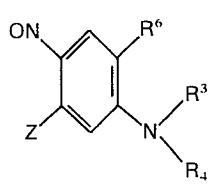

(2)

in welcher $R^3$, $R^4$, $R^6$ und Z die oben genannten Bedeutungen haben, mit einem m-Aminophenol der Formel (3)

(Fortsetzung nächste Seite)

EP 0 036 555 A1

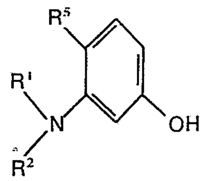

(3)

in welcher $R^1$, $R^2$ und $R^5$ die oben genannten Bedeutungen haben, in Gegenwart von Zinkchlorid und in Abwesenheit einer Base umsetzt. Die Zinkchloridkomplexverbindungen der Formel (1) können unter Abspaltung des Formelrestes Z oder unter oxidativer Entfernung dieses zum Stickstoffatom ortho-ständigen Formelrestes Z oder des anderen ortho-ständigen Wasserstoffatomes in die Phenoxazinfarbstoffe der Formel (4)

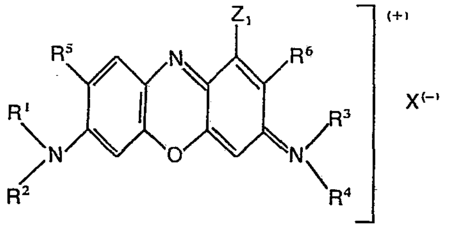

(4)

in welcher $R^1$, $R^2$, $R^3$, $R^4$, $R^5$ und $R^6$ die oben genannten Bedeutungen haben und $Z_1$ ein Halogenatom, eine niedere Alkylgruppe, eine Cyan-, Carbonsäure-, Carbamoyl-, Sulfamoyl-, eine durch niederes Alkyl mono- oder disubstituierte Carbamoyl- oder Sulfamoylgruppe oder eine Trifluormethyl- oder niedere Carbalkoxygruppe oder ein Wasserstoffatom bedeutet und $X^{(-)}$ für ein Anion steht, übergeführt werden.

0036555

HOECHST AKTIENGESELLSCHAFT HOE 80/F 059 Dr.ST

# Neue Zinkchloridkomplexverbindungen, Verfahren zu ihrer Herstellung sowie ihre Verwendung und Verfahren zur Herstellung von Phenoxazinverbindungen

Die vorliegende Erfindung liegt auf dem technischen Gebiet der Zwischenprodukte für Farbstoffe.

Aus der Europäischen Offenlegungsschrift Nr. 0005451 ist die Herstellung von chinoiden Schwermetallkomplexverbindungen durch Umsetzung von Nitrosierungsprodukten von oxäthylierten m-Aminophenolen mit einem m-Aminophenol in Gegenwart von Schwermetallsalzen und gegebenenfalls in Mischung mit einem basischen Metallsalz bekannt. Diese Schwermetallkomplexverbindungen lassen sich durch Lösen in einem wäßrigen neutralen oder alkalischen Medium in Phenoxazinverbindungen überführen.

Es wurde nun gefunden, daß es nicht erforderlich ist, zur Herstellung solcher Zwischenprodukte von oxäthylierten Nitroso-m-aminophenolen auszugehen.

Die vorliegende Erfindung betrifft neue Zinkchloridkomplexverbindungen, denen die Struktur der allgemeinen Formel (1)

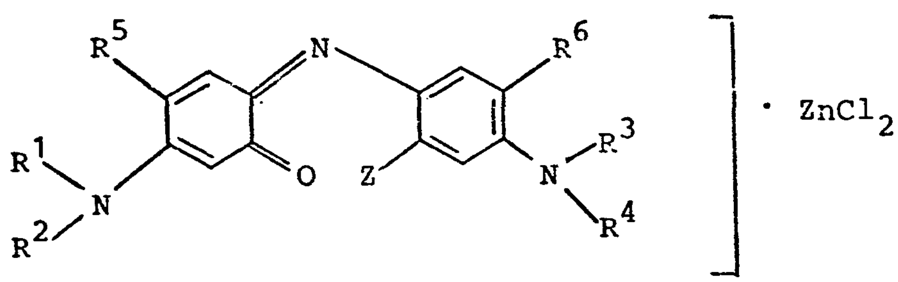

(1)

zugeordnet werden kann. In dieser allgemeinen Formel (1) bedeuten:

$R^1$ ist ein Wasserstoffatom oder eine niedere Alkylgruppe, die substituiert sein kann, beispielsweise durch Chlor,

eine Hydroxy-, niedere Alkoxy-, Cyan-, niedere Alkanoyloxy-, wie Acetyloxygruppe, Carbamoylgruppe oder einen Phenylrest;

$R^2$ ist ein Wasserstoffatom oder eine niedere Alkylgruppe, die substituiert sein kann, beispielsweise durch Chlor, eine Hydroxy-, niedere Alkoxy-, Cyan-, niedere Alkanoyloxy-, wie Acetyloxygruppe, Carbamoylgruppe oder einen Phenylrest, oder ist ein Phenylrest, der substituiert sein kann, beispielsweise durch Substituenten aus der Gruppe Chlor, niederes Alkyl, niederes Alkoxy, Carbamoyl, Sulfamoyl und durch niederes Alkyl mono- oder disubstituiertes Sulfamoyl oder Carbamoyl;

$R^3$ ist ein Wasserstoffatom oder eine niedere Alkylgruppe, die substituiert sein kann, beispielsweise durch Chlor, eine Hydroxy-, niedere Alkoxy-, Cyan-, niedere Alkanoyloxy-, wie Acetyloxygruppe, Carbamoylgruppe oder einen Phenylrest;

$R^4$ ist ein Wasserstoffatom oder eine niedere Alkylgruppe, die substituiert sein kann, beispielsweise durch Chlor, eine Hydroxy-, niedere Alkoxy-, Cyan-, niedere Alkanoyloxy-, wie Acetyloxygruppe, Carbamoylgruppe oder einen Phenylrest, oder ist ein Phenylrest, der substituiert sein kann, beispielsweise durch Substituenten aus der Gruppe Chlor, niederes Alkyl, niederes Alkoxy, Carbamoyl, Sulfamoyl und durch niederes Alkyl mono- oder disubstituiertes Sulfamoyl oder Carbamoyl;

$R^5$ ist ein Wasserstoffatom, ein Halogenatom, wie ein Chlor- oder ein Bromatom, eine niedere Alkylgruppe oder eine niedere Alkoxygruppe, oder

$R^1$ und $R^5$ bilden zusammen den o-Phenylenrest;

$R^6$ ist ein Wasserstoffatom, ein Halogenatom, wie ein Chlor- oder Bromatom, eine niedere Alkylgruppe oder eine niedere Alkoxygruppe;

Z ist ein Wasserstoffatom, ein Halogenatom, wie ein Chloratom, eine niedere Alkylgruppe, eine Cyan-, Carbonsäure-, Carbamoyl-, Sulfamoyl-, eine durch niederes Alkyl mono-

oder disubstituierte Carbamoyl- oder Sulfamoylgruppe, eine Trifluormethyl- oder niedere Carbalkoxygruppe, eine niedere Alkoxygruppe, die durch eine Cyangruppe substituiert sein kann, eine Acyloxygruppe, eine Acylaminogruppe, eine niedere Alkylaminogruppe oder eine niedere Dialkylaminogruppe.

Mit der vorstehenden oder auch nachfolgenden Angabe "niedere" werden bevorzugt solche Reste oder Verbindungen verstanden, in denen der Alkylrest jeweils 1 bis 4 C-Atome enthält. Die Acylreste in den oben erwähnten Acyloxy- und Acylaminogruppen sind solche von aliphatischen Carbonsäuren mit 1 bis 5 C-Atomen, wie beispielsweise der Propionsäure oder Essigsäure, oder einer aromatischen Carbonsäure, wie der Benzoesäure oder einer aromatischen Sulfonsäure, wie der Benzolsulfonsäure oder Toluolsulfonsäure. Niedere Alkylgruppen sind bevorzugt die Methyl- und Äthylgruppe, niedere Alkoxygruppen sind bevorzugt die Methoxy- und Äthoxygruppe. Niedere Carbalkoxygruppen sind bevorzugt die Carbomethoxy- und Carbäthoxygruppe.

Die vorliegende Erfindung betrifft weiterhin ein Verfahren zur Herstellung der obengenannten und definierten Verbindungen entsprechend der allgemeinen Formel (1), das dadurch gekennzeichnet ist, daß man eine Nitrosoverbindung der allgemeinen Formel (2)

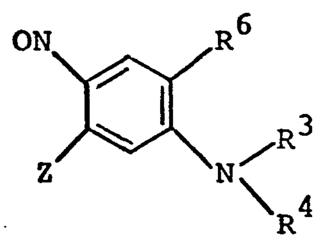

(2)

in welcher $R^3$, $R^4$, $R^6$ und Z die obengenannten Bedeutungen haben, mit einem m-Aminophenol der allgemeinen Formel (3)

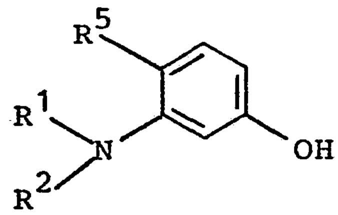

(3)

in welcher $R^1$, $R^2$ und $R^5$ die obengenannten Bedeutungen haben, in Gegenwart von Zinkchlorid und in Abwesenheit einer Base umsetzt. Die Reaktionspartner, d.h. die Verbindungen der Formel (2) und Formel (3) und Zinkchlorid, werden in äquimolaren Mengen eingesetzt; das Zinkchlorid kann auch in einem Überschuß, wie beispielsweise bis zu 1,5-molarem, vorzugsweise bis zu 1,2-molarem Überschuß, verwendet werden. Ebenso kann das Aminophenol der Formel (3) in bis zu 1,1-fach molarem Überschuß eingesetzt werden.

Die Nitrosoverbindung der allgemeinen Formel (2) wird in der Regel als freie Base, vorteilhaft als Zinkchlorid-Komplexverbindung eingesetzt.

Die Umsetzung der Reaktionspartner erfolgt in einem protischen oder aprotischen, polaren Lösungs- oder Verdünnungsmittel. Solche Lösungs- oder Verdünnungsmittel sind Wasser und wäßrige Lösungen von Elektrolyten, wie Natriumchlorid, Kaliumchlorid oder Natriumsulfat, sowie übliche protische oder aprotische, polare organische Lösungs- und Verdünnungsmittel, von denen die bevorzugt sind, die leicht und ohne Abwasserbelastung regenerierbar sind. Insbesondere bevorzugt sind solche organische polare Lösungs- oder Verdünnungsmittel, die mit Wasser teilweise oder ganz mischbar sind, wie niedere Alkanole, niedere Alkanglykole oder deren niederen Alkyläther, wie beispielsweise Methanol, Äthanol, Isopropanol, Butanol, Äthylenglykol, Äthylenglykol-monomethyläther, Äthylenglykol-monoäthyläther. In einigen Fällen, was durch einen Laborversuch leicht ermittelt werden kann, lassen sich jedoch als Lösungs- oder Verdünnungsmittel auch Formamid, Dimethylformamid, Dimethylsulfoxid, N-Methylpyrrolidon, Dioxan, Pyridin, Acetonitril, Essigsäureäthyl-

ester, Chloroform oder Chlorbenzol verwenden, natürlich auch Gemische von zwei oder mehreren all dieser genannten Lösungs- und Verdünnungsmittel. Soweit die oben erwähnten Lösungs- oder Verdünnungsmittel mit Wasser mischbar sind, können auch Gemische dieser Lösungs- und Verdünnungsmittel mit Wasser als Reaktionsmedium dienen.

Die erfindungsgemäße Umsetzung kann bei einer Temperatur zwischen 15°C und 150°C durchgeführt werden; bevorzugt arbeitet man bei einer Temperatur zwischen 55 und 95°C. Infolge der Anwesenheit der Zinkchlorids verläuft die Reaktion im schwach sauren Medium. Sofern Wasser als Reaktionsmedium zugegen ist, erfolgt die Umsetzung entsprechend bei einem pH-Wert von etwa 3,5 bis 7.

Die Bildung der Verbindungen der allgemeinen Formel (1) verläuft schnell und mit positiver Wärmetönung und ist bei etwa 80°C in wenigen Minuten beendet. Die tiefgefärbten Verbindungen der allgemeinen Formel (1) sind in der Regel, zumindest bei Raumtemperatur, in den obengenannten Lösungs- oder Verdünnungsmitteln nur wenig oder sehr schwer löslich, so daß sie sich gut kristallisiert und in hohen Ausbeuten praktisch analysenrein abscheiden.

Die m-Aminophenole der allgemeinen Formel (3), die als Reaktionspartner dienen, sollen zweckmäßig in dem verwendeten Lösungs- oder Verdünnungsmittel teilweise oder ganz löslich sein, so daß das Reaktionsmedium bezüglich dieser Verbindungen eine Lösung, eine feine Dispersion oder Suspension oder ein Gemisch derselben darstellt.

Das Zinkchlorid kann als wasserfreies Salz oder in Form einer Lösung in den Reaktionsansatz eingesetzt werden. Als Lösungen von Zinkchlorid werden vorzugsweise konzentrierte wäßrige Lösungen, wie beispielsweise etwa 40 bis 65 gew.-%ige wäßrige Lösungen, eingesetzt.

Die Nitrosoverbindungen der allgemeinen Formel (2), die ebenso wie die Verbindungen der allgemeinen Formel (3) seit langem bekannte und übliche Zwischenprodukte darstellen, lassen sich in üblicher Weise durch Nitrosierung der entsprechenden m-substituierten Anilinderivate herstellen. Diese Verfahrensweisen sind zahlreich in der Literatur beschrieben. Die Verbindungen der allgemeinen Formel (2) werden bevorzugt entweder als freie Base, insbesondere jedoch als deren Zinkchloridsalz, bevorzugt als Lösung oder Suspension in den oben erwähnten Lösungs- oder Verdünnungsmitteln oder Gemischen derselben *) Zweckmäßigerweise kann man die Isolierung der Nitrosoverbindung der allgemeinen Formel (2), insbesondere in trockener Form, auslassen und von den Lösungen oder Suspensionen der Nitrosoverbindungen, wie sie bei der Nitrosierung anfallen, ausgehen, wie beispielsweise von der Lösung oder Suspension des Hydrochlorids der Nitrosoverbindung in Wasser oder in wäßriger Elektrolytlösung. Diese salzsaure Lösung kann man mit einem basisch reagierenden Mittel, wie einem Alkalihydroxid oder -carbonat oder Ammoniak, beispielsweise mittels einer wäßrigen Natronlauge oder wäßrigen Ammoniaklösung, in die freie Nitrosobase überführen. Bevorzugt geht man jedoch vom Zinksalz dieser Nitrosoverbindung aus. Dieses läßt sich beispielsweise herstellen, indem man die oben erwähnte saure Lösung oder Suspension der Nitrosoverbindung mit wäßriger Natronlauge oder wäßriger Ammoniaklösung deutlich alkalisch stellt, beispielsweise auf einen pH-Wert von 9 bis 10, und sie sodann mit einer wäßrigen Zinkchloridlösung zusammengibt. Die so erhältliche Lösung, in der Regel Suspension, des Zinkchloridsalzes der Nitrosoverbindung der allgemeinen Formel (2) kann direkt in die Umsetzung mit dem Aminophenol der allgemeinen Formel (3) eingesetzt werden, ohne dieses Zinkchloridsalz der Nitrosoverbindung als Reaktionspartner erst isolieren zu müssen.

*) in die Reaktion eingesetzt.

Eine bevorzugte Ausführungsform ist deshalb die Umsetzung der Zinkchloridsalze der Nitrosoverbindung der allgemeinen

Formel (2) mit einem Aminophenol der allgemeinen Formel (3), bevorzugt in äquimolaren Mengen mit den obengenannten Lösungs- oder Verdünnungsmitteln oder Gemischen derselben als Reaktionsmedium.

Bevorzugt wird die Nitrosoverbindung in Lösung oder Suspension in einem der obengenannten Lösungs- oder Verdünnungsmittel oder Gemischen derselben zum Aminophenol, das in einem solchen Lösungs- oder Verdünnungsmittel oder Gemischen derselben teilweise oder gänzlich gelöst ist, zugegeben, wobei das Zinkchlorid als dritter Reaktionspartner entweder in der Lösung oder Suspension der Nitrosoverbindung oder in der Lösung (bzw. Lösung und Suspension) des Aminophenols oder in beiden vorliegen kann.

Das erfindungsgemäße Verfahren gestaltet sich auch dann besonders einfach, wenn man eine Nitrosoverbindung der allgemeinen Formel (2), beispielsweise in trockener Form, mit einer Aminophenolverbindung der allgemeinen Formel (3) und wasserfreiem Zinkchlorid in äquimolarem Verhältnis oder praktisch äquimolarem Verhältnis in den genannten Lösungs- oder Verdünnungsmitteln, wie vorzugsweise niederen Alkanolen, Äthylenglykol oder deren niederen Alkyläthern, bei einer Temperatur bis zu vorzugsweise 95°C, beispielsweise bei 70 bis 90°C, und bevorzugt unter Rühren miteinander umsetzt. Der gebildete Zinkchloridkomplex scheidet sich bald kristallin aus. Nach kurzer Zeit ist dünnschichtchromatographisch keine Nitrosoverbindung mehr nachweisbar. Man kühlt den Reaktionsansatz sodann ab und isoliert den Niederschlag; die Ausbeute ist fast quantitativ.

Von den obigen Lösungs- oder Verdünnungsmitteln werden bevorzugt Methanol, Äthanol, Äthylenglykol, Äthylenglykol-monomethyläther oder Äthylenglykol-monoäthyläther und Isopropanol oder Gemische derselben, ebenso diese Lösungs- oder Verdünnungsmittel in Gemischen mit Wasser verwendet.

Die nach Beendigung der Reaktion ausgefallenen Verbindungen der allgemeinen Formel (1) werden abgesaugt und gegebenenfalls mit Wasser oder einer wäßrigen Elektrolytlösung oder mit einem anderen der obengenannten Verdünnungs- oder Lösungsmittel oder Gemischen derselben, in denen die Substanz schwer löslich ist, gewaschen. Die Ausbeute ist fast quantitativ, die Produkte sind sehr rein.

Die Verbindungen der allgemeinen Formel (1) bzw. die nach dem erfindungsgemäßen Verfahren durch Umsetzung der Verbindungen der allgemeinen Formel (2) mit den Verbindungen der allgemeinen Formel (3) und Zinkchlorid erhältlichen Zinkchloridkomplexverbindungen stellen wertvolle Zwischenprodukte dar; sie können zur Herstellung von Phenoxazinverbindungen (Farbstoffen) dienen. Die vorliegende Erfindung betrifft somit auch die Verwendung der Verbindungen der allgemeinen Formel (1) bzw. dieser erfindungsgemäß erhältlichen Zinkchloridkomplexverbindungen als Zwischenprodukte zur Herstellung von Phenoxazinverbindungen der allgemeinen Formel (4)

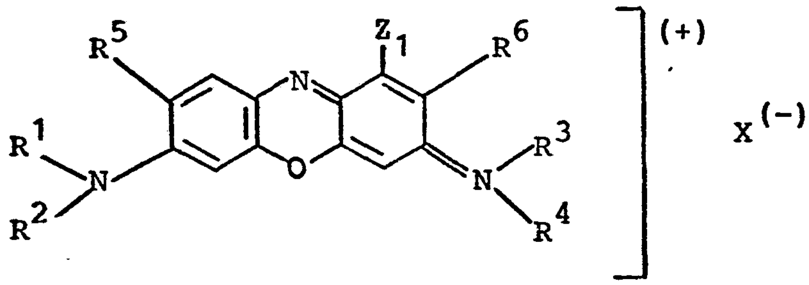

(4)

in welcher $R^1$, $R^2$, $R^3$, $R^4$, $R^5$ und $R^6$ die obengenannten Bedeutungen haben, $Z_1$ ein Halogenatom, eine niedere Alkylgruppe, eine Cyan-, eine Carbonsäure-, eine Carbamoyl-, Sulfamoyl-, eine durch niederes Alkyl mono- oder disubstituierte Carbamoyl- oder Sulfamoylgruppe, eine Trifluormethyl- oder niedere Carbalkoxygruppe, bevorzugt ein Wasserstoffatom bedeutet und $X^{(-)}$ für ein Anion steht, bzw. betrift ein Verfahren zur Herstellung dieser Phenoxazinverbindungen der allgemeinen Formel (4) durch Ringschluß der erfindungsgemäßen Zinkchloridkomplexverbindungen entsprechend der allgemeinen Formel (1) zum Phenoxazinring.

Die Zinkchloridkomplexverbindungen entsprechend der allgemeinen Formel (1), in welchen Z für eine niedere Alkoxygruppe, die durch eine Cyangruppe substituiert sein kann, eine Acyloxygruppe, eine Acylaminogruppe, eine niedere Alkylaminogruppe oder eine niedere Dialkylaminogruppe steht, gehen unter Abspaltung dieser Gruppe Z und Ringschluß in die Phenoxazinverbindung über. Hierzu behandelt man diese Verbindungen der allgemeinen Formel (1) mit dieser spezifisch genannten Gruppe Z in einem geeigneten protischen oder aprotischen, polaren Lösungsmittel oder Verdünnungsmittel oder Mischungen derselben, gegebenenfalls unter Erwärmen,+) und gegebenenfalls in Gegenwart einer Säure oder einer Base und/oder gegebenenfalls in Gegenwart einer Verbindung, die das komplexgebundene Zink aus der Verbindung der allgemeinen Formel (1) als schwerlösliche Zinkverbindung abscheidet oder dieses in ein lösliches*) Zinkat überführt. Bei diesen Maßnahmen findet Ringschluß der Chinoniminverbindung unter Abspaltung des Restes Z zur Phenoxazinverbindung statt. Lösungs- oder Verdünnungsmittel, die zur Behandlung der Verbindung der allgemeinen Formel (1) zwecks Ringschluß dienen können, sind beispielsweise Wasser oder wäßrige Elektrolytlösungen oder ein organisches Lösungs- oder Verdünnungsmittel. Vorzugsweise behandelt man die Verbindungen der allgemeinen Formel (1) in diesen Lösungs- oder Verdünnungsmitteln unter Erwärmen, beispielsweise bei einer Temperatur zwischen 30 und 100°C. Hierbei kann man vorteilhaft anorganische oder organische Säuren oder anorganische oder organische basische Verbindungen, einschließlich der sauren Salze oder basischen Salze von anorganischen oder organischen Säuren, zugeben, um einen pH-Wert zwischen 2 und 14, vorzugsweise 5 und 9, einzustellen. Vorteilhaft verfährt man in der Weise, daß man das Zink der Verbindung der allgemeinen Formel (1) in Form einer unlöslichen Zinkverbindung abscheidet oder in eine lösliche Zinkverbindung, wie beispielsweise in das Zinkatanion, überführt.

+) bspw. bei einer Temperatur von etwa 5 bis 120°C,

*) Zinksalz oder

Zinkabscheidungsmittel bzw. Zinkfällungsmittel sind alle diejenigen Agentien, die mit dem Zink eine unlösliche Verbindung, wie insbesondere Zinkphosphat und Zinkcarbonat, bilden. Verbindungen, die als solche Fällungsmittel dienen können, sind beispielsweise die Alkalimetallcarbonate oder -hydrogencarbonate, wie Natriumcarbonat, Kaliumcarbonat, Natriumbicarbonat, Kaliumbicarbonat, aber auch Ammoniumcarbonat, Calciumcarbonat, Calciumbicarbonat, Magnesiumcarbonat, des weiteren die Alkalimetallsalze der Phosphorsäure, wie Mono-, Di- und Trinatriumphosphat und die entsprechenden Kaliumphosphate und Ammoniumphosphate, des weiteren die Alkali- und Ammoniumsalze der Oxalsäure.

Verbindungen, die das Zink in eine lösliche Komplexverbindung überführen, sind beispielsweise die Alkalimetallsalze der Äthylendiamintetraessigsäure oder der Nitrilotriessigsäure, Ammoniak, Mono-, Di- und Triäthanolamin, Äthylendiamin, Piperidin und Pyridin sowie alkalische Laugen, wie Alkalihydroxid.

Besonders vorteilhaft geht man in der Weise vor, daß man die Verbindungen der allgemeinen Formel (1) mit Wasser vermischt und ein Alkalimetall- oder Ammoniumcarbonat oder -bicarbonat oder ein Alkalimetall- oder Ammoniumphosphat, wie beispielsweise Natriumcarbonat, Natriumbicarbonat, Ammoniumcarbonat, Dinatriumhydrogenphosphat oder Diammoniumhydrogenphosphat, zugibt oder wäßrige Lösungen solcher Verbindungen mit den Verbindungen der allgemeinen Formel (1) vermischt. Die erhältliche wäßrige Suspension oder Anschlämmung wird sodann erwärmt. Hierbei beginnt sich die Lösung tiefblau zu färben, und die Bildung der blauen Phenoxazinverbindung ist nach kurzer Zeit beendet. Man saugt von der ausgefällten, wasserunlöslichen Zinkverbindung (Carbonat, Phosphat) ab.

Die so erhaltene wäßrige Lösung des Phenoxazins kann auf übliche Weise aufgearbeitet werden, so beispielsweise in das

Chlorid durch Ansäuern mit Salzsäure auf einen pH-Wert von 5 bis 6 überführt werden. Das Salz der Phenoxazinverbindung kann dann in üblicher Weise isoliert werden, beispielsweise durch Sprühtrocknen oder durch Ausfällung als Chlorzinkdoppelsalz mit Zinkchlorid und Natriumchlorid.

Verbindungen entsprechend der allgemeinen Formel (1), in welchen Z für ein Halogenatom, eine niedere Alkylgruppe, eine Cyan-, Carbonsäure-, Carbamoyl-, Sulfamoyl-, eine durch niederes Alkyl mono- oder disubstituierte Carbamoyl- oder Sulfamoylgruppe, eine Trifluormethyl- oder eine niedere Carbalkoxygruppe, bevorzugt jedoch für ein Wasserstoffatom steht, lassen sich durch oxidativen Ringschluß in die entsprechenden Phenoxazinfarbstoffe überführen. Als Oxidationsmittel zum oxidativen Ringschluß dieser Zinkchloridkomplexverbindungen entsprechend der allgemeinen Formel (1), die eine andere Gruppe Z als die erwähnte abspaltbare Gruppe Z (Alkoxy, Acyloxy, Acylamino, Alkylamino) enthalten, dienen vorteilhaft salpetrige Säure (beispielsweise im Reaktionsansatz in an und für sich üblicher Weise hergestellt durch Zugabe von Natriumnitrit zur sauren Reaktionslösung) oder Peressigsäure. Als Lösungsmittel für diesen oxidativen Ringschluß sind Wasser und gegen diese Oxidationsmittel inerte organische Lösungsmittel geeignet, wie beispielsweise niedere Alkanole, wie Methanol oder Äthanol, oder Essigsäure. Der oxidative Ringschluß wird in saurem Reaktionsmedium durchgeführt, bevorzugt bei einem pH-Wert zwischen 2 und 6; die Umsetzung kann bei niedriger und auch bei erhöhter Temperatur erfolgen, wie beispielsweise bei einer Temperatur zwischen 10 und 80°C. Erforderlich bei der oxidativen Ringschlußreaktion ist die Anwesenheit einer Verbindung, die das in der Zinkverbindung der allgemeinen Formel (1) komplexgebundene Zink aus dieser Verbindung zu entfernen vermag, beispielsweise durch Fällung des Zinks als unlösliche Verbindung oder durch Überführung des Zinks in eine andere, *) löslichen Komplex durch ein komplexierendes Agenz. Solche

*) lösliche Verbindung, wie einen

zinkfällenden oder komplexbildenden Agentien sind zuvor schon bei der Ringschlußreaktion unter Abspaltung der Gruppe Z beschrieben worden.

Die auf diese Weise erhältlichen Phenoxazinverbindungen können, wie oben beschrieben, aus dem Reaktionsansatz des oxidativen Ringschlusses aufgearbeitet und isoliert werden.

Die so erhältlichen bekannten Phenoxazinverbindungen haben die bekannten Phenoxazinfarbstoffeigenschaften mit jedoch oft reinerer Nuance. Sie können in üblicher und bekannter Weise als Farbstoffe zum Färben von den hierfür bekannten Fasermaterialien, insbesondere von Polyacrylnitrilfasern, dienen, wie in der Literatur zahlreich beschrieben.

Die nachstehenden Beispiele dienen zur Erläuterung der Erfindung. Die Teile sind Gewichtsteile, die Prozentangaben beziehen sich auf Gewichtsprozente, sofern nicht anders vermerkt. Gewichtsteile und Volumenteile bestehen im Verhältnis von Kilogramm zu Liter.

Beispiel 1

12,3 Teile 2-Amino-4-hydroxy-toluol, 16,0 Teile Zinkchlorid und 19,9 Teile 4-Nitroso-3-methoxy-diäthylanilin werden in 100 Volumenteilen Äthanol 2 Stunden unter Rückfluß gerührt. Nachdem dünnschichtchromatographisch keine Nitrosoverbindung mehr nachweisbar ist, kühlt man das Reaktionsgemisch ab, saugt den dunklen kristallinen Niederschlag ab, wäscht ihn mit 100 Volumenteilen Äthanol und trocknet ihn bei 60°C. Man erhält 43,6 Teile eines sehr schwer löslichen, grünglänzenden, fast schwarzen Kristallpulvers, dem die Formel

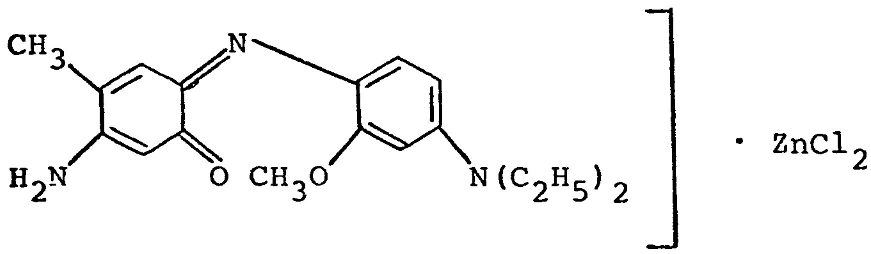

zugeordnet wird.
Die Analyse, berechnet auf Mol 449,4 bei $C_{18}H_{23}N_3O_2 \cdot ZnCl_2$, ergab folgende Werte:

| | | | | |
|---|---|---|---|---|
| ber.: | C | 48,06 %, | gef.: | 47,8 %, |
| | H | 5,12 %, | | 5,3 %, |
| | N | 9,35 %, | | 9,4 %, |
| | O | 7,12 %, | | 7,5 %, |
| | $OCH_3$ | 6,90 %, | | 6,8 %, |
| | Zn | 14,55 %, | | 14,3 %, |
| | Cl | 15,80 %, | | 16,0 %. |

Beispiel 2

Die in Beispiel 1 beschriebene Zinkkomplexverbindung läßt sich vorteilhaft ohne Isolierung der Nitrosoverbindung herstellen. Man kann hierbei folgendermaßen vorgehen:
Man löst 179 Teile 3-Methoxy-diäthylanilin in einer Mischung aus 600 Volumenteilen einer gesättigten wäßrigen Natriumchloridlösung und 232 Volumenteilen einer wäßrigen 30 %igen

Salzsäure. Bei 0 bis 5°C läßt man 139 Volumenteile einer wäßrigen 40 %igen Natriumnitritlösung unter gutem Rühren langsam einlaufen; man rührt die erhaltene dicke, gelbe Suspension des Nitrosohydrochlorids bei deutlichem Nitritüberschuß nach. Sodann zerstört man den Nitritüberschuß mit wenig Amidosulfonsäure. Man stellt bei 15 bis 20°C alkalisch, beispielsweise mit 25 %iger wäßriger Ammoniaklösung auf einen pH-Wert von 9. Zu dieser hierbei gebildeten Suspension der Nitrosobase tropft man bei 15 bis 20°C 141 Volumenteile einer wäßrigen 64 %igen Zinkchlorid-Lösung ein, wobei das*) Zinkchlorid-Doppelsalz der Nitrosoverbindung kristallin ausfällt. Der pH zeigt einen Wert von 5,6 bis 6,1.

*) orange gefärbte

Diese Kristallsuspension wird in eine siedende Lösung von 123 Teilen 2-Amino-4-hydroxy-toluol in 600 Volumenteilen Äthanol gegossen. Man heizt das Reaktionsgemisch auf Siedetemperatur. Nach etwa 15 Minuten ist die Umsetzung beendet. Es hat sich ein grober Kristallniederschlag ausgeschieden. Man kühlt das Reaktionsgemisch auf 40°C ab, saugt ab und wäscht mit etwa 1000 Teilen kaltem Wasser nach. Nach dem Trocknen erhält man 436 Teile der in Beispiel 1 formelmäßig angegebenen Verbindung in grünglänzenden, fast schwarzen Kristallen.

## Beispiel 3

Man verfährt wie im Beispiel 2 angegeben, ersetzt jedoch dort das 2-Amino-4-hydroxy-toluol durch 165 Teile 3-Diäthylaminophenol. Es werden 460 Teile einer Verbindung erhalten, der die Formel

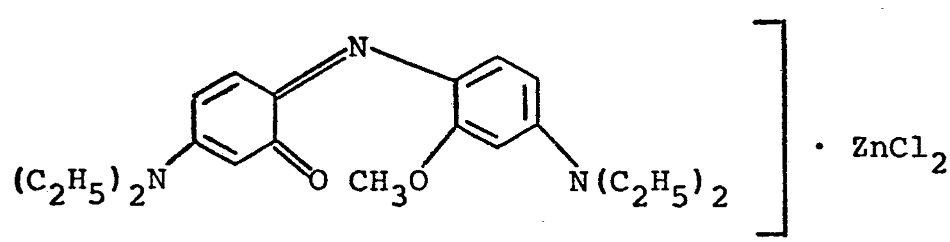

zugeordnet wird. Sie liegt in Form von grünglänzenden, fast schwarzen Kristallen vor.

Analyse (ber. auf Mol 491,3 bei $C_{21}H_{29}N_3O_2 \cdot ZnCl_2$):

| | | | | |
|---|---|---|---|---|
| ber.: | C | 51,29 %, | gef.: | 51,2 %, |
| | H | 5,90 %, | | 5,8 %, |
| | N | 8,55 %, | | 8,6 %, |
| | O | 6,51 % | | 6,6 %, |
| | $OCH_3$ | 6,31 %, | | 6,2 %, |
| | Zn | 13,29 %, | | 13,2 %, |
| | Cl | 14,45 %, | | 14,5 %. |

Beispiel 4

12,3 Teile 2-Amino-4-hydroxy-toluol, 16,0 Teile Zinkchlorid und 19,7 Teile 4-Nitroso-diphenylamin werden in 400 Volumenteilen Äthanol angerührt und anschließend 2 Stunden bei Siedetemperatur erhitzt. Die gelbe Farbe der Nitrosoverbindung verschwindet, und es beginnt ein dunkler Niederschlag auszufallen. Man kühlt auf 50°C ab, saugt das Produkt ab, wäscht es mit 100 Volumenteilen Äthanol nach und trocknet es bei 60°C. Es werden 42,9 Teile einer Verbindung in Form eines fast schwarzen Kristallpulvers erhalten, der die Formel

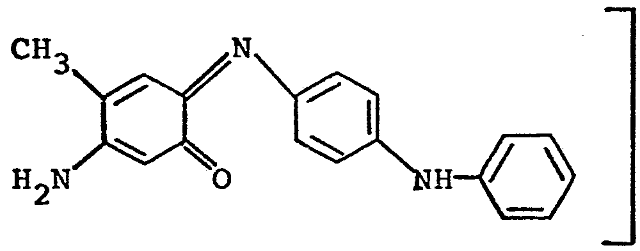 · $ZnCl_2$

zugeordnet wird.

Analyse (ber. auf Mol 439,3 bei $C_{19}H_{17}N_3O \cdot ZnCl_2$):

| | | | | |
|---|---|---|---|---|
| ber.: | C | 51,90 %, | gef.: | 51,6 %, |
| | H | 3,87 %, | | 4,0 %, |
| | N | 9,56 %, | | 9,5 %, |
| | O | 3,64 %, | | 3,8 %, |
| | Zn | 14,86 %, | | 14,7 %, |
| | Cl | 16,16 %, | | 15,8 %. |

Beispiel 5

16,5 Teile 3-Diäthylaminophenol, 26 Teile Zinkchlorid und 17,8 Teile 4-Nitroso-diäthylanilin werden in 100 Volumenteilen Methylglykol 2 Stunden lang bei 80°C gerührt. Danach ist dünnschichtchromatographisch keine Nitrosoverbindung mehr zu erkennen. Das Reaktionsgemisch wird auf 20°C abge-

kühlt, der fast schwarze Niederschlag abgesaugt, mit 100 Volumenteilen Äthanol gewaschen und bei 60°C getrocknet. Es werden 45,8 Teile einer Verbindung erhalten, der die Formel

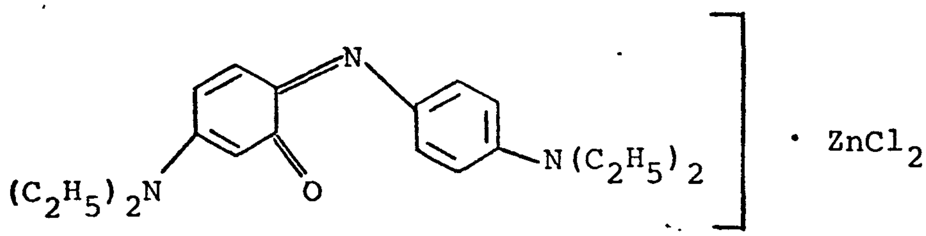

zugeordnet wird.

Analyse (ber. auf Mol 461,3 bei $C_{20}H_{27}N_3O \cdot ZnCl_2$)

| | | | | |
|---|---|---|---|---|
| ber.: | C | 52,03 %, | gef.: | 51,8 %, |
| | H | 5,85 %, | | 6,0 %, |
| | N | 9,10 %, | | 9,0 %, |
| | O | 3,47 %, | | 3,8 %, |
| | Zn | 14,16 %, | | 13,9 %, |
| | Cl | 15,39 %, | | 15,5 %. |

Die obengenannte neue Zinkchloridkomplexverbindung wird in etwa gleich guter Ausbeute erhalten, wenn man es gemäß der in Beispiel 2 beschriebenen Verfahrensvariante herstellt.

Beispiel 6

16,5 Teile 3-Diäthylaminophenol, 16 Teile Zinkchlorid und 19,2 Teile 4-Nitroso-3-methyl-N,N-diäthylanilin werden 3 Stunden in 200 Volumenteilen Methanol bei Siedetemperatur gerührt. Anschließend wird der Reaktionsansatz abgekühlt, das ausgeschiedene Produkt abgesaugt, mit 100 Volumenteilen Methanol gewaschen und getrocknet. Es werden 46,5 Teile einer Verbindung erhalten, der die Formel

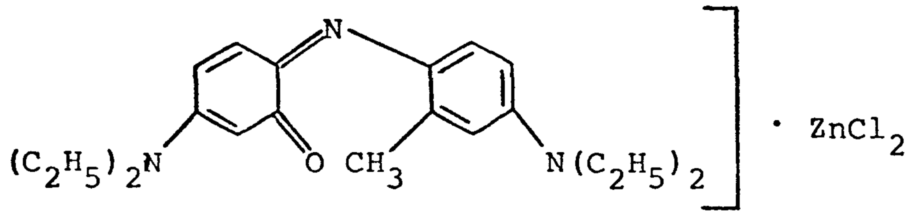

zugeordnet wird.

Analyse (ber. auf Mol 475,3 bei $C_{21}H_{29}N_3O \cdot ZnCl_2$):

| | | | | |
|---|---|---|---|---|
| ber.: | C | 53,02 %, | gef.: | 53,0 %, |
| | H | 6,10 %, | | 6,4 %, |
| | N | 8,84 %, | | 8,7 %, |
| | O | 3,37 %, | | 4,1 %, |
| | Zn | 13,74 %, | | 13,5 %, |
| | Cl | 14,94 %, | | 15,0 %. |

Die hier hergestellte neue Zinkkomplexverbindung wird in gleich guter Ausbeute und Qualität erhalten, wenn man sie analog der in Beispiel 2 beschriebenen Verfahrensvariante herstellt.

Beispiel 7

Verfährt man wie in Beispiel 6 oder in analoger Weise wie in Beispiel 2 beschrieben, setzt jedoch als Ausgangsverbindungen das 3-Hydroxy-carbazol und 4-Nitroso-3-methoxy-diäthylanilin bzw. 3-Methoxy-diäthylanilin (zwecks Nitrosierung) ein, so erhält man dunkle Kristalle einer Verbindung entsprechend der Formel

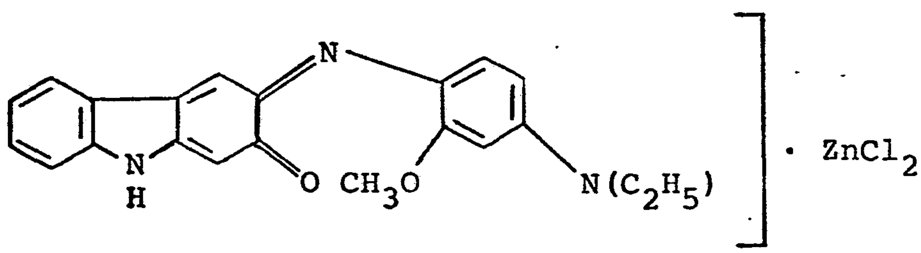

Beispiel 8

19,9 Teile 3-Hydroxy-4'-methyl-diphenylamin, 13,0 Teile Zinkchlorid und 28,1 Teile 4-Nitroso-3-äthoxy-diäthylanilin werden in 200 Volumenteilen Äthylglykol 2 Stunden lang bei 80°C gerührt. Nach 2 Stunden ist die Umsetzung beendet. Man saugt den unlöslichen Niederschlag ab, wäscht ihn mit 200 Volumenteilen Methanol und trocknet ihn bei 60°C.

Man erhält 53 Teile eines dunklen Kristallpulvers einer Verbindung, der die Formel

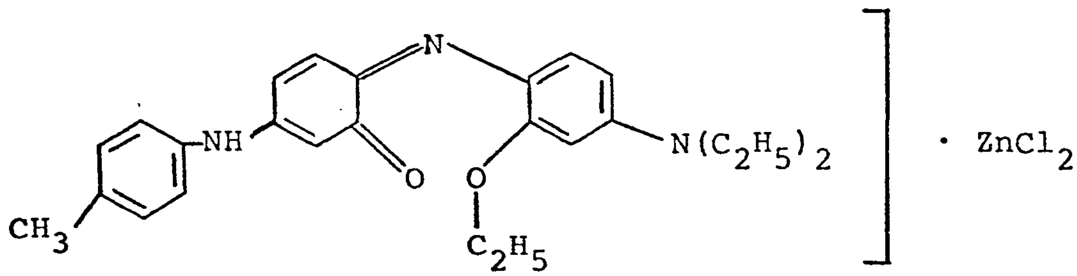

zugeordnet wird.

Analyse (ber. auf Mol 539,4 bei $C_{25}H_{21}N_3O_2 \cdot ZnCl_2$):

| | | | | |
|---|---|---|---|---|
| ber.: | C | 55,62 %, | gef.: | 55,3 %, |
| | H | 5,38 %, | | 5,8 %, |
| | N | 7,79 %, | | 7,8 %, |
| | O | 5,93 %, | | 6,0 %, |
| | $OC_2H_5$ | 8,34 %, | | 8,1 %, |
| | Zn | 12,12 %, | | 11,8 %, |
| | Cl | 13,16 %, | | 12,9 %. |

Diese Zinkchloridkomplexverbindung kann in gleich guter Ausbeute und Reinheit erhalten werden, wenn man sie analog der in Beispiel 2 beschriebenen Verfahrensvariante herstellt.

Beispiel 9

15,8 Teile 2-Äthylamino-4-hydroxy-toluol, 20,0 Teile Zinkchlorid und 19,7 Teile 4-Nitroso-diphenylamin werden in 200 Volumenteilen Äthanol 2 Stunden zum Sieden erhitzt. Man saugt den ausgefallenen Niederschlag ab, wäscht ihn mit 100 Volumenteilen Äthanol und trocknet ihn. Es werden 45,5 Teile einer Verbindung in Form grünglänzender, fast schwarzer Kristalle erhalten, der die Formel

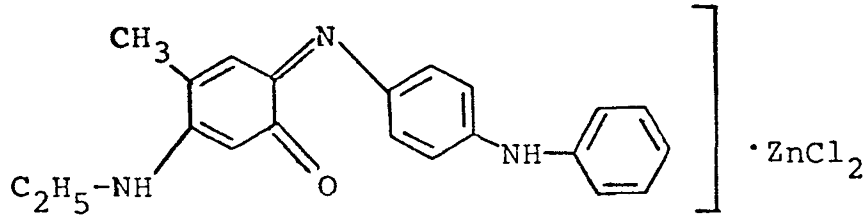

0036555

zugeordnet wird.

<u>Analyse</u> (ber. auf Mol 467,3 bei $C_{21}H_{21}N_3O \cdot ZnCl_2$):

| | | | | |
|---|---|---|---|---|
| ber.: | C | 53,93 %, | gef.: | 53,6 %, |
| | H | 5,14 %, | | 4,6 %, |
| | N | 8,99 %, | | 8,9 %, |
| | O | 3,42 %, | | 3,4 %, |
| | Zn | 13,97 %, | | 14,1 %, |
| | Cl | 15,19 %, | | 15,5 %. |

Verfährt man analog zu der in Beispiel 2 beschriebenen Verfahrensweise, setzt jedoch äquivalente Mengen an dem 2-Äthylamino-4-hydroxytoluol und Diphenylamin ein, so erhält man die hier hergestellte Zinkchloridkomplexverbindung in etwa gleich guter Ausbeute und Reinheit.

<u>Beispiel 10</u>

19,9 Teile 3-Hydroxy-4'-methyl-diphenylamin, 26,0 Teile Zinkchlorid und 13,2 Teile 4-Nitroso-3-methoxy-diäthylanilin werden in 200 Volumenteilen Äthanol 2 Stunden bei Siedetemperatur gerührt. Es fällt ein dunkler Niederschlag aus, der nach Abkühlen auf 60°C abgesaugt wird; man wäscht ihn mit 100 Volumenteilen Äthanol nach und trocknet ihn bei 60°C. Es werden 50 Teile einer Verbindung in Form dunkler Kristalle erhalten, der die Formel

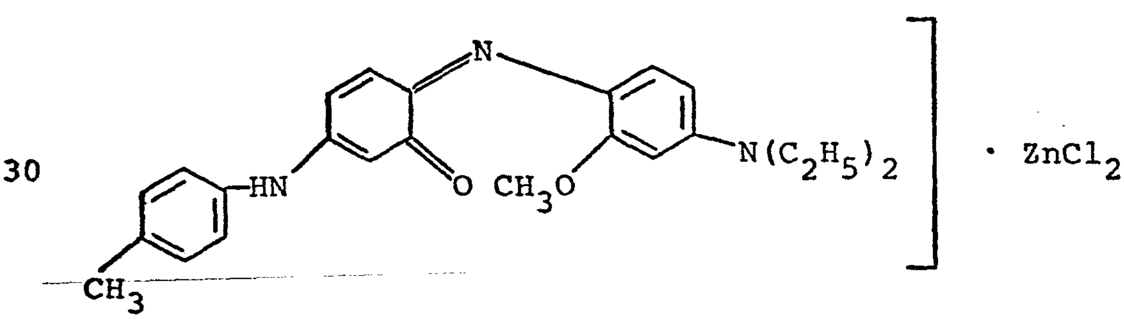

zugeordnet wird.

Analyse (ber. auf Mol 525,4 bei $C_{24}H_{27}N_3O_2 \cdot ZnCl_2$):

| | | | | |
|---|---|---|---|---|
| ber.: | C | 54,82 %, | gef.: | 54,5 %, |
| | H | 5,14 %, | | 5,4 %, |
| | N | 7,99 %, | | 7,8 %, |
| | O | 6,09 %, | | 6,2 %, |
| | $OCH_3$ | 5,9 %, | | 5,7 %, |
| | Zn | 12,45 %, | | 12,2 %, |
| | Cl | 13,51 %, | | 13,0 %. |

In gleich guter Ausbeute und Reinheit erhält man die hier hergestellte Zinkchloridkomplexverbindung, wenn man sie analog der in Beispiel 2 beschriebenen Verfahrensweise herstellt.

Beispiel 11

16,5 Teile 3-Diäthylaminophenol, 26,0 Teile Zinkchlorid und 24,2 Teile 4-Nitroso-3-methoxy-4'-methyl-diphenylamin werden 2 Stunden lang in 200 Volumenteilen Methylglykol bei 80°C gerührt. Man kühlt das Reaktionsgemisch danach auf 40°C ab, saugt den ausgefallenen dunklen Niederschlag ab, wäscht ihn mit 100 Volumenteilen Methanol und trocknet ihn bei 60°C. Es werden 51,5 Teile eines fast schwarzen Kristallpulvers der Verbindung entsprechend der Formel

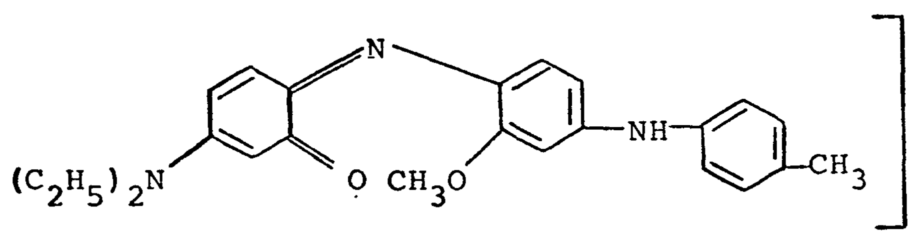 $\cdot ZnCl_2$

erhalten.

Analyse (ber. auf Mol 525,4 bei $C_{24}H_{27}N_3O_2 \cdot ZnCl_2$):

| | | | | |
|---|---|---|---|---|
| ber.: | C | 54,82 %, | gef.: | 54,4 %, |
| | H | 5,14 %, | | 5,3 %, |
| | N | 7,99 %, | | 8,1 %, |
| | O | 6,09 %, | | 6,2 %, |
| | $OCH_3$ | 5,90 %, | | 6,1 %, |

| | | | | |
|---|---|---|---|---|
| ber.: | Zn | 12,45 %, | gef.: | 12,3 %, |
| | Cl | 13,51 %, | | 13,5 %. |

Diese Zinkchloridkomplexverbindung wird in etwa gleicher Ausbeute und Qualität erhalten, wenn man sie analog der Verfahrensweise des Beispieles 2 synthetisiert.

Beispiel 12

16,5 Teile 3-Diäthylaminophenol, 16,0 Teile Zinkchlorid und 25,6 Teile 4-Nitroso-3-äthoxy-4'-methyl-diphenylamin werden in 200 Volumenteilen Isopropanol 2 Stunden lang bei Siedetemperatur gerührt. Wenn nach etwa 2 Stunden keine Nitrosoverbindung dünnschichtchromatographisch mehr zu erkennen ist, wird das ausgeschiedene Reaktionsprodukt bei 50°C abgesaugt, mit 200 Volumenteilen Isopropanol gewaschen und bei 60°C getrocknet. Es werden 52,9 Teile einer Verbindung in Form grünlich glänzender Kristalle erhalten, der die Formel

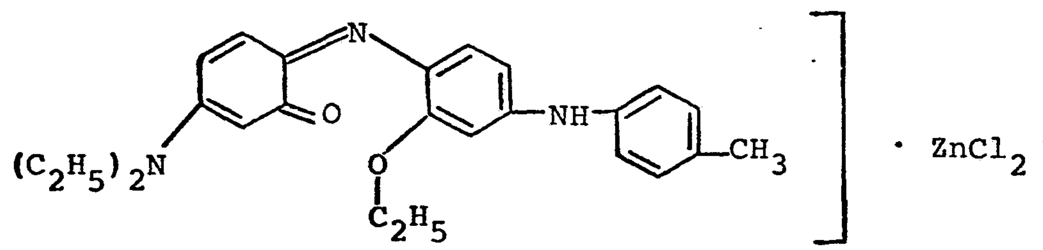

zugeordnet wird.

Analyse (ber. auf Mol 539,4 bei $C_{25}H_{29}N_3O_2 \cdot ZnCl_2$):

| | | | | |
|---|---|---|---|---|
| ber.: | C | 55,62 %, | gef.: | 55,6 %, |
| | H | 5,38 %, | | 5,3 %, |
| | N | 7,79 %, | | 7,9 %, |
| | O | 5,93 %, | | 6,2 %, |
| | $OC_2H_5$ | 8,34 %, | | 8,5 %, |
| | Zn | 12,12 %, | | 11,9 %, |
| | Cl | 13,16 %, | | 12,9 %. |

Man verfährt analog der in Beispiel 2 beschriebenen Verfah-

0036555

rensweise, setzt jedoch eine äquivalente Menge an 3-Diäthylaminophenol und zur Nitrosierung eine äquivalente Menge an 3-Äthoxy-4'-methyl-diphenylamin ein. Die so hergestellte Zinkchloridkomplexverbindung entspricht in Ausbeute und Reinheit der obengenannten Zinkchloridkomplexverbindung.

Beispiele 13 bis 72

Verfährt man in erfindungsgemäßer Weise zur Herstellung der Zinkchloridkomplexverbindungen entsprechend der allgemeinen Formel (1), beispielsweise in analoger Weise zu den Verfahrensvarianten, die in den vorherigen Beispielen 1 bis 12 beschrieben sind, und geht hierbei von anderen Ausgangsverbindungen entsprechend der allgemeinen Formeln (2) und (3) bzw. der allgemeinen Formel (3) und zur Herstellung der Nitrosoverbindung von einem entsprechenden Amin der allgemeinen Formel

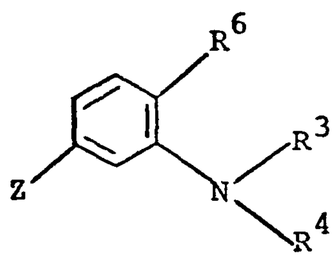

in welcher $R^3$, $R^4$, $R^6$ und Z die obengenannten Bedeutungen haben, aus, so beispielsweise von den in den nachfolgenden Tabellenbeispielen durch die Angabe der Substituenten charakterisierten Ausgangsverbindungen, so erhält man entsprechend die durch die Formelreste charakterisierten Verbindungen (1) mit den angegebenen Farbtönen in einer guten Ausbeute.

| Bsp. | Verbindung (1) aus | | | | | | | Farbe der Verbindung (1) |
|---|---|---|---|---|---|---|---|---|
| | Verbindung (2) | | | | | Verbindung (3) | | |
| | $R^1$ | $R^2$ | $R^5$ | Z | $R^3$ | $R^4$ | $R^6$ | |
| 13 | $CH_3$ | $CH_3$ | H | $C_2H_5O$ | $C_2H_5$ | $C_2H_5$ | H | fast schwarz |
| 14 | $CH_3$ | $C_2H_5$ | $CH_3$ | $CH_3O$ | $C_2H_5$ | $C_2H_5$ | H | dito |
| 15 | H | $CH_2CH_2CN$ | $CH_3$ | $CH_3O$ | $C_2H_5$ | $C_2H_5$ | H | dito |
| 16 | $CH_3$ | $CH_3$ | H | $CH_3O$ | H | o-Methyl-phenyl | H | dito |
| 17 | $C_2H_5$ | $C_2H_5$ | H | $CH_3O$ | H | o-Methyl-phenyl | H | dito |
| 18 | $C_2H_5$ | H | $CH_3$ | $CH_3O$ | H | o-Methyl-phenyl | H | dito |
| 19 | H | H | $CH_3$ | $C_2H_5O$ | H | o-Methyl-phenyl | H | dito |
| 20 | $C_2H_5$ | $C_2H_5$ | H | $CH_3O$ | H | m-Methyl-phenyl | H | dito |
| 21 | $CH_3$ | $C_2H_5$ | $CH_3$ | $CH_3O$ | H | Phenyl | H | dito |
| 22 | $CH_3$ | $CH_3$ | H | $CH_3O$ | H | p-Methoxy-phenyl | H | dito |
| 23 | $C_2H_5$ | $C_2H_5$ | H | $CH_3O$ | H | $CH_2CH_2CN$ | $CH_3O$ | dito |
| 24 | H | H | $CH_3$ | $CH_3O$ | H | $CH_2CH_2CN$ | $CH_3O$ | dito |
| 25 | $C_2H_5$ | H | $CH_3$ | $C_2H_5O$ | H | $CH_2CH_2CN$ | $C_2H_5O$ | dito |
| 26 | $CH_3$ | $CH_3$ | H | $C_2H_5O$ | H | $CH_2CH_2CN$ | $C_2H_5O$ | dito |
| 27 | $CH_3$ | $CH_3$ | H | $CH_3O$ | H | p-Äthoxy-phenyl | H | dito |
| 28 | $C_2H_5$ | $C_2H_5$ | H | $C_2H_5O$ | H | p-Äthoxy-phenyl | H | dito |
| 29 | $C_2H_5$ | $C_2H_5$ | H | H | $CH_3$ | p-Äthoxy-phenyl | H | dito |

| Bsp. | Verbindung (1) aus: Verbindung (2) $R^1$ | Verbindung (2) $R^2$ | $R^5$ | Z | Verbindung (3) $R^3$ | Verbindung (3) $R^4$ | Verbindung (3) $R^6$ | Farbe der Verbindung (1) |
|---|---|---|---|---|---|---|---|---|
| 30 | $C_2H_5$ | $C_2H_5$ | H | $CH_3COO$ | $C_2H_5$ | $C_2H_5$ | H | fast schwarz |
| 31 | $C_2H_5$ | $C_2H_5$ | H | $CH_3CONH$ | $C_2H_5$ | $C_2H_5$ | H | dito |
| 32 | $C_2H_5$ | $C_2H_5$ | H | Dimethyl-sulfamoyl | $C_2H_5$ | $C_2H_5$ | H | dito |
| 33 | $C_2H_5$ | $C_2H_5$ | H | COOH | $CH_3$ | $CH_3$ | H | dito |
| 34 | $C_2H_5$ | $C_2H_5$ | H | Cl | $C_2H_5$ | $C_2H_5$ | H | dito |
| 35 | $C_2H_5$ | $C_2H_5$ | H | $CH_3CONH$ | $CH_2CH_2OH$ | $CH_2CH_2OH$ | $CH_3O$ | dito |
| 36 | $C_2H_5$ | $C_2H_5$ | H | $CH_3CONH$ | $CH_2CH_2OH$ | $CH_2CH_2CN$ | $CH_3O$ | dito |
| 37 | H | $C_2H_5$ | H | $CH_3COO$ | $C_2H_5$ | $C_2H_5$ | H | dito |
| 38 | H | H | $CH_3$ | $CH_3CONH$ | $CH_2CH_2OH$ | $CH_2CH_2OH$ | $CH_3O$ | dito |
| 39 | $C_2H_5$ | $C_2H_5$ | H | $CH_3$ | $CH_3COOCH_2CH_2-$ | $CH_3COOCH_2CH_2-$ | H | dito |
| 40 | H | $C_2H_5$ | H | Cl | $CH_2CH_2OH$ | $CH_2CH_2OH$ | H | dito |
| 41 | H | $C_2H_5$ | $CH_3$ | $CH_3O$ | H | o-Methoxy-phenyl | H | dito |
| 42 | H | $C_2H_5$ | $CH_3$ | $CH_3O$ | H | o-Äthoxy-phenyl | H | dito |
| 43 | H | $C_2H_5$ | $CH_3$ | $CH_3O$ | H | p-Chlor-phenyl | H | dito |
| 44 | H | H | $CH_3$ | $CH_3O$ | H | o-Methyl-phenyl | H | dito |
| 45 | $CH_3$ | $C_2H_5$ | $CH_3$ | $CH_3O$ | H | o-Methyl-phenyl | H | dito |
| 46 | H | H | $CH_3$ | $CH_3COO$ | $C_2H_5$ | $C_2H_5$ | H | dito |

| | Verbindung (1) aus | | | | | | | Farbe der Verbindung (1) |
|---|---|---|---|---|---|---|---|---|
| | Verbindung (2) | | | | | Verbindung (3) | | |
| Bsp. | $R^1$ | $R^2$ | $R^5$ | Z | $R^3$ | $R^4$ | $R^6$ | |
| 47 | $C_2H_5$ | $C_2H_5$ | H | H | $C_2H_5$ | $CH_2CH_2CONH_2$ | H | fast schwarz |
| 48 | H | Phenyl | H | $CH_3O$ | H | Phenyl | H | dito |
| 49 | $C_2H_5$ | $C_2H_5$ | H | $CN-CH_2-O-$ | $C_2H_5$ | $C_2H_5$ | H | dito |
| 50 | H | o-Äthoxy-phenyl | H | $CH_3O$ | $C_2H_5$ | $C_2H_5$ | H | dito |
| 51 | H | o-Äthoxy-phenyl | H | $C_2H_5O$ | $C_2H_5$ | $C_2H_5$ | H | dito |
| 52 | $C_2H_5$ | $C_2H_5$ | H | $CH_3O$ | H | o-Äthoxy-phenyl | H | dito |
| 53 | $C_2H_5$ | $C_2H_5$ | H | $C_2H_5O$ | H | o-Äthoxy-phenyl | H | dito |
| 54 | H | o-Äthoxy-phenyl | H | $CH_3COO$ | $C_2H_5$ | $C_2H_5$ | H | dito |
| 55 | H | H | H | $CH_3O$ | $C_2H_5$ | $C_2H_5$ | H | dito |
| 56 | H | H | $CH_3$ | $C_2H_5O$ | $C_2H_5$ | $C_2H_5$ | H | dito |
| 57 | H | $C_2H_5$ | $CH_3$ | $CH_3O$ | $C_2H_5$ | $C_2H_5$ | H | dito |
| 58 | H | $C_2H_5$ | $CH_3$ | $C_2H_5O$ | $C_2H_5$ | $C_2H_5$ | H | dito |
| 59 | $C_2H_5$ | $C_2H_5$ | H | $C_2H_5O$ | $C_2H_5$ | $C_2H_5$ | H | dito |
| 60 | H | Phenyl | H | $C_2H_5O$ | $C_2H_5$ | $C_2H_5$ | H | dito |
| 61 | H | Phenyl | H | $CH_3O$ | $C_2H_5$ | $C_2H_5$ | H | dito |
| 62 | H | Phenyl | H | $CH_3COO$ | $C_2H_5$ | $C_2H_5$ | H | dito |
| 63 | $C_2H_5$ | $C_2H_5$ | H | $CH_3O$ | H | Phenyl | H | dito |
| 64 | $C_2H_5$ | $C_2H_5$ | H | $C_2H_5O$ | H | Phenyl | H | dito |
| 65 | $C_2H_5$ | $C_2H_5$ | H | $CH_3O$ | H | 2,5-Dimethyl-phenyl | H | dito |

| Bsp. | Verbindung (2) $R^1$ | Verbindung (2) $R^2$ | Verbindung (1) aus $R^5$ | Z | $R^3$ | Verbindung (3) $R^4$ | Verbindung (3) $R^6$ | Farbe der Verbindung (1) |
|---|---|---|---|---|---|---|---|---|
| 66 | $C_2H_5$ | $C_2H_5$ | H | $C_2H_5O$ | H | 2,5-Dimethyl-phenyl | H | fast schwarz |
| 67 | H | 2,5-Dimethyl-phenyl | H | $CH_3O$ | $C_2H_5$ | $C_2H_5$ | H | dito |
| 68 | H | dito | H | $C_2H_5O$ | $C_2H_5$ | $C_2H_5$ | H | dito |
| 69 | H | dito | H | $CH_3COO$ | $C_2H_5$ | $C_2H_5$ | H | dito |
| 70 | H | p-Methoxy-phenyl | H | $CH_3O$ | $C_2H_5$ | $C_2H_5$ | H | dito |
| 71 | H | $C_2H_5$ | $CH_3$ | $C_2H_5O$ | H | o-Methyl-phenyl | H | dito |
| 72 | H | p-Methyl-phenyl | H | $CH_3COO$ | $C_2H_5$ | $C_2H_5$ | H | dito |

0036555

Beispiel 73

27,8 Teile der Zinkchloridkomplexverbindung von Beispiel 50 werden in 100 Volumenteilen N-Methyl-pyrrolidon angeschlämmt. Man erwärmt auf 40°C und rührt bei dieser Temperatur etwa 8 Stunden. Es bildet sich eine tiefblaue Lösung. Wenn dünnschichtchromatographisch nur der reinblaue Flecken des zu bildenden Phenoxazinfarbstoffes zu sehen ist, filtriert man von einer geringen Menge einer Verunreinigung ab und hat eine gebrauchsfertige Lösung des Chlorides bzw. Chlorzinksalzes des Farbstoffkations der Formel

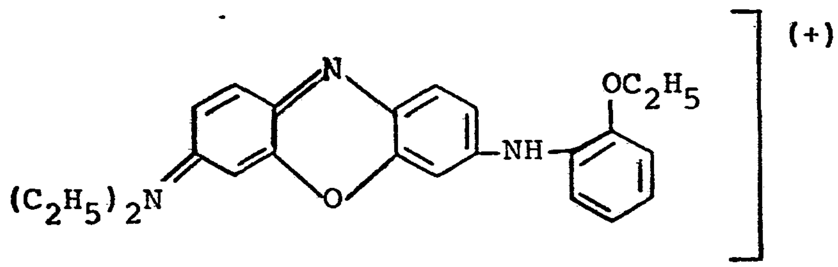

Will man eine zinkfreie Ware isolieren, so tropft man die blaue Lösung in eine Mischung von 500 Volumenteilen Wasser und 8 Teilen Diammoniumhydrogenphosphat und filtriert nach etwa einer Stunde vom ausgefallenen Zinkphosphat ab. Man gibt 5 Teile 5n-Salzsäure hinzu und salzt mit 50 Teilen Natriumchlorid das Farbstoffchlorid aus.
Mit diesen Phenoxazinfarbstoffsalzen läßt sich Polyacrylnitrilfasermaterial in üblicher Weise in reinen blauen, farbkräftigen und echten Tönen färben.

Zu dem gleichen Farbstoff gelangt man, wenn man bei sonst gleicher Arbeitsweise die Zinkchloridkomplexverbindungen von den Beispielen 51, 52, 53 oder 54 einsetzt.

Beispiel 74

Man stellt eine alkalische wäßrige Lösung von einem pH-Wert von 9 mittels 23,1 Teilen 85 %iger wäßriger Phosphorsäure und etwa 48 Teilen 33 %iger Natronlauge in 300 Teilen Wasser her. Zu dieser gibt man bei etwa 20°C 87,1 Teile der Zinkchloridkomplexverbindung von Beispiel 55 und rührt diese

Suspension etwa 12 Stunden bei 20°C. Der pH-Wert ist danach auf etwa 6,7 gefallen. Man erwärmt das Reaktionsgemisch auf 40°C und rührt weitere 2 Stunden nach, wobei der pH-Wert auf etwa 6,1 fällt. Dann erwärmt man auf 60°C und rührt für 3 Stunden bei dieser Temperatur, wonach ein pH-Wert von 4,9 erhalten wird. In einer dünnschichtchromatographischen Probe sieht man dann nur einen rein blauen Fleck neben einer Spur einer Verunreinigung. Man saugt ab und wäscht den Zinkphosphatrückstand in Portionen mit 300 Teilen Wasser von 60°C nach. Aus der klaren, blauen Farbstofflösung fällt man mit 15 Teilen Zinkchlorid und 100 Teilen Natriumchlorid das Chlorzinksalz des Farbstoffes der Formel

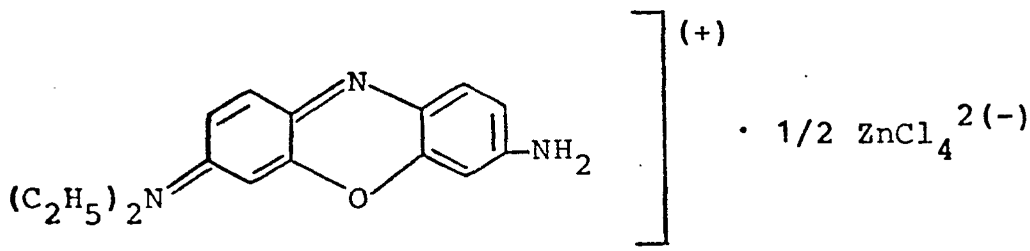

aus, saugt ihn ab und trocknet ihn bei 60°C. Man erhält 74 Teile eines Farbstoffpulvers, das in Wasser sehr gut löslich ist.

Eine zinkfreie Farbstofflösung kann man in üblicher Weise durch Fällung des Zinks als Phosphat erhalten.

<u>Beispiel 75</u>

89,9 Teile der gemäß Beispiel 1 oder Beispiel 2 hergestellten Zinkchloridkomplexverbindung der im Beispiel 1 angegebenen Formel werden in 200 Teilen Wasser von 60°C angeschlämmt und im Verlauf von etwa 2 Stunden mit 24 Teilen Natriumhydrogencarbonat portionsweise versetzt. Die Reaktionsmischung färbt sich tiefblau; man rührt noch etwa 3 Stunden bei 60°C nach, wobei der pH-Wert auf etwa 7 abfällt. Man saugt das Zinkcarbonat ab, wäscht es mit Wasser von 60°C und erhält eine klare, dunkelblaue Lösung des Farbstoffes der Formel

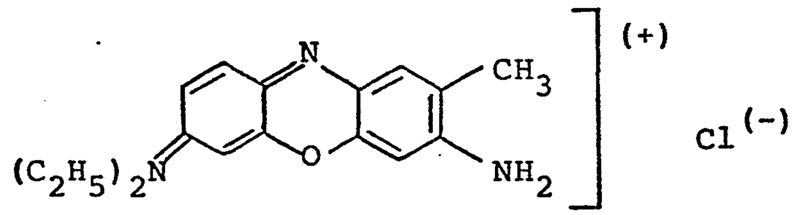

die, mit Eisessig auf einen pH-Wert von 4,5 eingestellt, eine gebrauchsfertige Flüssigeinstellung darstellt. Die Ausbeute bei der Ringschlußreaktion zum Phenoxazin ist quantitativ, was durch colorimetrische Farbstärkemessung ermittelt werden kann.

Der Farbstoff kann auf übliche Weise mittels einer wäßrigen Zinkchloridlösung und Natriumchlorid in das Tetrachlorzinksalz übergeführt und quantitativ in groben, grünschimmernden Kristallen ausgefällt werden. Es ist in Wasser und verdünnter Essigsäure sehr leicht löslich und färbt aus saurem Bad Polyacrylnitrilfasermaterial in üblicher Weise in kräftigen, reinen blauen Tönen mit guten Echtheiten.

Beispiel 76

92,6 Teile der Zinkchloridkomplexverbindung von Beispiel 56 werden in 250 Teilen Wasser von 60°C angeschlämmt. Unter gutem Rühren werden innerhalb von 3 Stunden 30 Teile Diammoniumhydrogenphosphat eingestreut. Man rührt weitere 3 Stunden bei 60°C und filtriert sodann die erhaltene tiefblaue Lösung mit einem pH-Wert von 6 bis 6,4 vom Zinkphosphat ab, das mit 250 Teilen Wasser von 60°C nachgewaschen werden kann. Man erhält so eine gebrauchsfertige Flüssigeinstellung des in Beispiel 75 formelmäßig angegebenen Farbstoffchlorides. Eine colorimetrische Farbstärkemessung zeigt, daß die Ringschlußreaktion zum Phenoxazin praktisch quantitativ abgelaufen ist. Der Zinkgehalt dieser Farbstofflösung liegt unter 10 ppm, so daß eine Verwendung des Farbstoffes auch in solchen Fällen möglich ist, wo die handelsüblichen Tetrachlorzinkate nicht eingesetzt werden können. Aus dieser Farbstofflösung kann man in üblicher Weise durch

Sprühtrocknung das Farbstoffchlorid, vermischt mit einer geringen Menge an Ammoniumchlorid in gut kristallisierten, grün glänzenden Kristallen in quantitativer Ausbeute erhalten. Es ist in Wasser sehr gut löslich, ebenso in niederen Alkoholen, wie Methanol und Äthanol, in Äthylenglykol, in Säureamiden, wie Dimethylformamid oder N-Methylpyrrolidon, oder in aliphatischen Carbonsäuren, wie Ameisensäure, Essigsäure oder Propionsäure.

Wie mit dem nach Beispiel 75 hergestellten Farbstoffsalz kann man mit dem hier hergestellten Phenoxazinfarbstoff Polyacrylnitrilfasermaterialien in üblicher Weise in reinen blauen Tönen mit hoher Farbstärke färben.

Beispiel 77

95,5 Teile der Zinkchloridkomplexverbindung von Beispiel 57 werden in 300 Teilen Wasser von 60°C angeschlämmt. Im Verlauf von etwa 3 Stunden trägt man portionsweise 25 Teile Ammoniumcarbonat ein. Man rührt noch 3 Stunden bei 60°C nach. Dünnschichtchromatographisch ist im Reaktionsansatz danach nur noch die gebildete Phenoxazinverbindung neben einer Spur einer Verunreinigung zu erkennen. Man saugt vom Zinkcarbonat ab, wäscht es mit 250 Teilen Wasser von 60°C und erhält ein klares blaues Filtrat mit einem pH-Wert von 7,5. Mit Eisessig wird der pH auf 5,8 gestellt. Man erhält eine gebrauchsfertige Lösung des Phenoxazinfarbstoffes der Formel

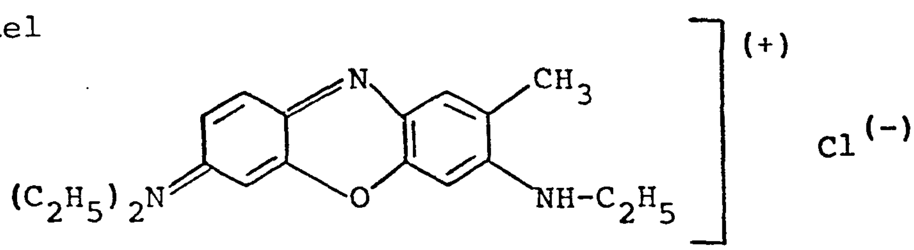

mit der Polyacrylnitrilfasermaterialien in üblicher Weise in reinen blauen, farbstarken Tönen mit guten Echtheiten gefärbt werden kann.

Eine colorimetrische Farbstärkemessung des erhaltenen Filtrats zeigt, daß die Ringschlußreaktion praktisch quantitativ verlaufen ist.

Beispiel 77a

Man verfährt wie in Beispiel 77 beschrieben, geht jedoch von 97 Teilen der Zinkchloridkomplexverbindung von Beispiel 58 aus. Man erhält den Phenoxazinfarbstoff in gleich hoher Ausbeute und Qualität.

Beispiel 78

Eine Lösung mit einem pH-Wert von 9 aus 23,1 Teilen einer 85 %igen wäßrigen Phosphorsäure, etwa 48 Teilen 33 %iger Natronlauge und 300 Teilen Wasser werden bei 20°C mit 94 Teilen der Zinkchloridkomplexverbindung von Beispiel 3 versetzt. Man rührt 12 Stunden lang, wobei der pH-Wert auf 7,8 abfällt. Die Temperatur wird sodann auf 40°C erhöht; es wird weitere 2 Stunden gerührt, wonach die Reaktionslösung einen pH-Wert von 7,3 zeigt. Anschließend rührt man noch 3 Stunden bei 60°C bis zu einem pH von 6 bis 6,4. Man erhält eine tiefblaue Lösung der in quantitativer Ausbeute gebildeten Phenoxazinverbindung. Man saugt vom Zinkphosphat ab. Der Zinkgehalt des Filtrats liegt unter 10 ppm. Durch Sprühtrocknung kann man in üblicher Weise das Farbstoffchlorid entsprechend der Formel

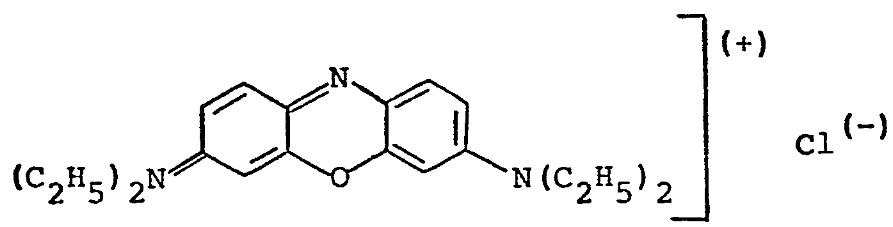

erhalten. Die Lösung kann auch mittels Natriumborfluorid in üblicher Weise in das sehr gut kristallisierte Farbstofftetrafluorborat übergeführt werden.

Diese aus der Zinkchloridkomplexverbindung von Beispiel 3 erhaltenen Farbstoffsalze färben Polyacrylnitrilfasermaterialien in reinen blauen Tönen mit hoher Farbstärke und in guten Echtheiten.

Beispiel 79

Zu einer alkalischen Natriumphosphatlösung, wie sie am Anfang des Beispieles 78 beschrieben ist, gibt man bei 20°C 96 Teile der Zinkchloridkomplexverbindung von Beispiel 59 und rührt zuerst 12 Stunden lang bei 20°C, sodann 2 Stunden bei 40°C und sodann noch 3 Stunden bei 60°C. Man erhält eine dunkelblaue Lösung von einem pH-Wert von 6,5, der die gebildete Phenoxazinverbindung in quantitativer Ausbeute enthält. Man saugt vom Zinkphosphat ab, wäscht mit 200 Teilen Wasser von 60°C, gibt zu dem Filtrat 12 Teile einer wäßrigen, 65 %igen Zinkchloridlösung und fällt das Farbstoffsalz mit etwa 100 Teilen Natriumchlorid vollständig aus. Man erhält auf diese Weise das Tetrachlorozink-Salz des in Beispiel 78 angegebenen Phenoxazinfarbstoffes. Mit ihm lassen sich ebenfalls reine blaue Färbungen auf Polyacrylnitrilfasermaterial mit hoher Farbstärke erhalten.

Beispiel 80

Zu einer Lösung von 115,5 Teilen einer 85 %igen wäßrigen Phosphorsäure in 1500 Teilen Wasser gibt man langsam etwa 239 Teile einer 33 %igen Natronlauge hinzu und erhält so eine alkalische wäßrige Lösung mit einem pH-Wert von 9. Bei einer Temperatur von 20°C trägt man 491 Teile der Zinkchloridkomplexverbindung von Beispiel 3 zusammen mit 50 Teilen Kieselgur ein, rührt den Reaktionsansatz 12 Stunden bei 20°C, 2 Stunden bei 40°C und schließlich*) bei 60°C. Man erhält eine tiefblaue Lösung mit einem pH-Wert von 6,3. Dünnschichtchromatographisch ist nur ein reinblauer Fleck zu erkennen. Man saugt ab, wäscht mit 1000 Teilen Wasser von 60°C nach und erhält eine tiefblaue, reine Farbstofflösung, die entsprechend einer colorimetrischen Farbstärkemessung das Phenoxazin-Chlorid entsprechend der in Beispiel 78 angegebenen Formel in praktisch quantitativer Ausbeute enthält. Das Farbstoffsalz kann durch Eindampfen der Lösung isoliert werden. Man erhält einen Rückstand von etwa 520 Teilen. Man kann das Farbstoffsalz in 300 Teilen Äthylen-

*) drei Stunden

glykol lösen, wobei 38 Teile Natriumchlorid zurückbleiben, von dem man abfiltriert. Man erhält so eine konzentrierte Lösung der in Beispiel 78 formelmäßig angegebenen Phenoxazinchlorid-Verbindung in Äthylenglykol. Mit ihr lassen sich in üblicher Weise Färbebäder ansetzen, mit denen Polyacrylnitrilfasermaterial in hoher Farbstärke in reinen *) blauen Tönen gefärbt werden kann. *) grünstichig

Beispiel 81

26 Teile der Zinkchloridkomplexverbindung von Beispiel 60 werden in einem Gemisch von 300 Teilen Wasser und 400 Volumenteilen Toluol suspendiert. Man tropft 100 Volumenteile einer wäßrigen 5n-Natronlauge im Verlauf von einer Stunde zu und rührt sodann 2 Stunden bei 50°C. Die Toluolschicht färbt sich intensiv bordorot, die Wasserphase wird fast farblos. Man trennt die wäßrige Phase ab, wäscht die Toluolphase noch zweimal mit jeweils 100 Teilen Wasser aus und treibt anschließend das Toluol mit Wasserdampf ab. Man erhält nach dem Erkalten 17 Teile grünglänzender Kristalle der Anhydrobase der Formel

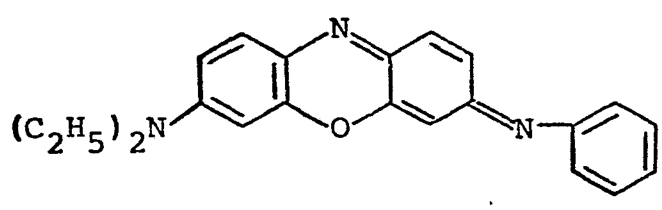

Diese löst sich mit Säuren zum grünstichig blauen Phenoxazinsalz, das nach üblichen Färbemethoden Polyacrylnitrilfasermaterialien in farbstarken Tönen mit reiner grünstichig blauer Farbe färbt. -

Die hier hergestellte Anhydrobase kann in gleicher Weise erhalten werden, wenn man von den Zinkchloridkomplexverbindungen von Beispiel 61, 62, 63 oder 64 ausgeht und in der hier angegebenen Verfahrensweise arbeitet.

Beispiel 82

27 Teile der Zinkchloridkomplexverbindung von Beispiel 65 werden in 100 Teilen Wasser bei 30°C angerührt. Man gibt im Verlauf von 1 Stunde 50 Volumenteile einer wäßrigen 25 %igen Ammoniaklösung hinzu, rührt erst bei 30°C 2 Stunden und sodann bei 50°C weitere 5 Stunden. Man läßt erkalten, saugt den dunklen Niederschlag ab, wäscht ihn mit Wasser neutral und trocknet ihn bei 60°C. Man erhält 18,5 Teile dunkler, grünglänzender Kristalle der Anhydrobase der Formel

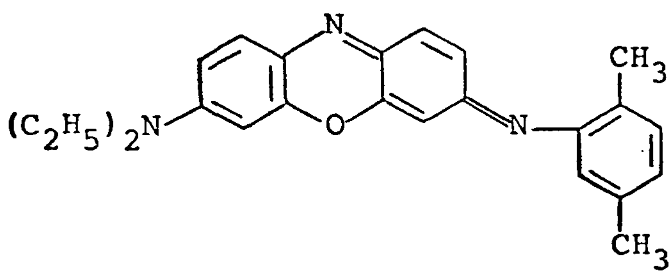

die dünnschichtchromatographisch einheitlich ist. Mit Säuren erhält man das Phenoxazinfarbstoffsalz, das in üblicher Weise zum Färben von Polyacrylnitrilfasermaterialien eingesetzt werden kann und diese Materialien in reinen grünstichigblauen Tönen färbt. -

Die hier hergestellte Anhydrobase kann in gleicher Qualität und Ausbeute nach der angegebenen Verfahrensweise erhalten werden, wenn man von der Zinkchloridkomplexverbindung von Beispiel 66, 67, 68 oder 69 ausgeht.

Beispiel 83

50,9 Teile der Zinkchloridkomplexverbindung von Beispiel 7 werden in 150 Volumenteilen Dimethylformamid suspendiert. Man rührt 8 bis 10 Stunden bei 40°C und anschließend noch 12 Stunden bei 20°C. Dünnschichtchromatographisch zeigt die erhaltene tiefblaue Lösung neben einer geringen Verunreinigung und wenig Ausgangsmaterial die erhaltene reinblaue Phenoxazinverbindung. Zu dieser Reaktionslösung gibt

man 500 Teile Wasser und 300 Volumenteile einer 5n-Salzsäure hinzu, erwärmt auf 80°C, saugt ab und wäscht mit etwa 1000 Teilen Wasser von 80°C nach. Mit 12 Volumenteilen einer wäßrigen 65 %igen Zinkchloridlösung und 100 Teilen Natriumchlorid wird das Chlorzinksalz des Phenoxazinfarbstoffes ausgefällt. Man saugt es ab und trocknet bei 60°C.
Man erhält 40 Teile des Farbstoffes der Formel

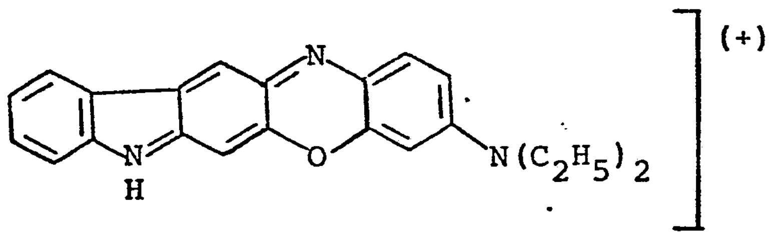

· 1/2 $ZnCl_4^{2(-)}$

in fast schwarzen Kristallen. Mit ihm kann man in bekannter Weise Polyacrylnitrilfasermaterialien färben und erhält farbstarke violettblaue Färbungen von guten Echtheiten. -

Diesen Farbstoff kann man in gleicher Ausbeute und Qualität nach der angegebenen Verfahrensweise erhalten, wenn man von 52 Teilen der Zinkchloridkomplexverbindung der Formel

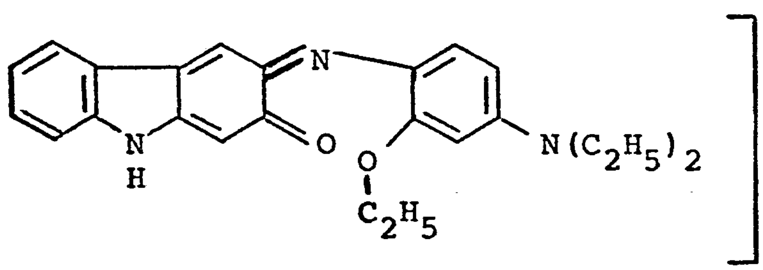

· $ZnCl_2$

ausgeht.

Beispiel 84

27 Teile der Zinkchloridkomplexverbindung von Beispiel 70 werden in 200 Volumenteilen Äthanol suspendiert. Im Verlauf von einer Stunde gibt man bei 25°C 40 Volumenteile einer wäßrigen 5n-Natronlauge hinzu. Die Lösung färbt sich rotviolett, und es beginnt ein heller Niederschlag aus Natriumchlorid und Zinkhydroxid auszufallen. Man rührt noch 12 Stunden bei 25°C nach, saugt ab und versetzt das Filtrat unter gutem Rühren tropfenweise mit einer Mischung aus

500 Teilen Wasser, 50 Volumenteilen einer 5n-Salzsäure und 50 Teilen Natriumchlorid. Den Niederschlag saugt man ab und trocknet ihn. Man erhält 20,5 Teile der Verbindung der Formel

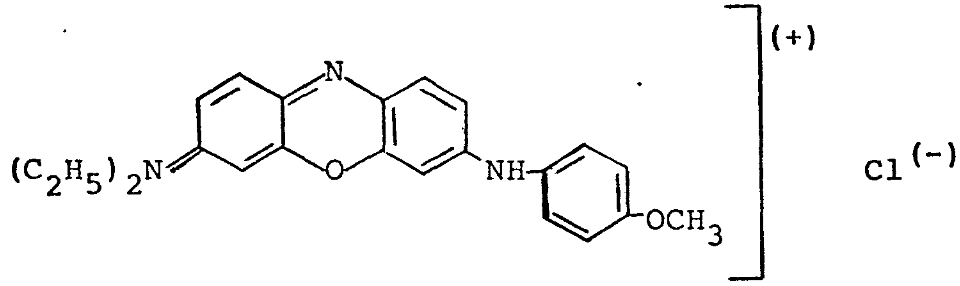

in Form dunkler, grünschimmernder Kristalle. Dieser Phenoxazinfarbstoff färbt in bekannter Weise Polyacrylnitrilfasermaterialien in farbtiefen, reinen grünstichig blauen Tönen mit sehr guter Lichtechtheit.

Beispiel 85

25,5 Teile der Zinkchloridkomplexverbindung von Beispiel 18 werden in 150 Teilen einer 10 %igen wäßrigen Ammoniaklösung 20 Stunden lang bei 60°C gerührt. Man saugt danach ab, wäscht den Rückstand mit 200 Teilen Wasser neutral und trocknet bei 60°C. Es werden 16,9 Teile der Verbindung der Formel

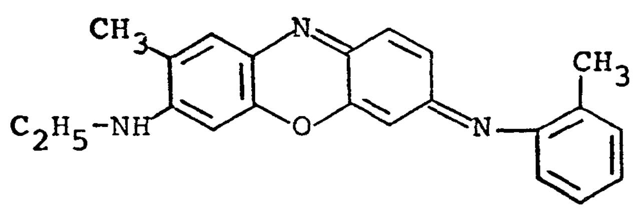

in Form eines dunklen Kristallpulvers erhalten. Diese Anhydrobase ist praktisch zinkfrei. Sie löst sich in Säuren unter Bildung des blauen Phenoxazinfarbstoffsalzes, das nach bekannten Färbemethoden Polyacrylnitrilfasermaterialien in reinen blauen, farbstarken Tönen färbt. - Den Phenoxazinfarbstoff erhält man in gleicher Qualität und Ausbeute nach der hier beschriebenen Verfahrensweise, wenn man von der Zinkchloridkomplexverbindung von Beispiel 71 ausgeht.

Beispiel 86

26,2 Teile der Zinkchloridkomplexverbindung von Beispiel 10 werden in 100 Teilen Wasser suspendiert. Man gibt innerhalb von 30 Minuten 75 Teile Triäthanolamin hinzu und rührt noch 20 Stunden bei 50°C. Man saugt ab und wäscht mit Wasser neutral. Nach Trocknung der Substanz erhält man 17 Teile der zinkfreien Anhydrobase der Formel

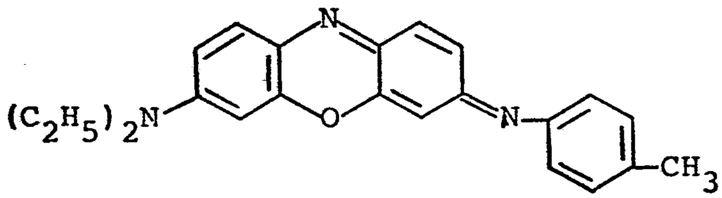

in Form dunkler, grünschimmernder Kristalle. Mit Säuren liefert sie das blaue Phenoxazinfarbstoffsalz, das in üblicher Färbeweise Polyacrylnitrilfasermaterialien in reinen blauen Tönen mit guten Echtheiten und in hoher Farbstärke liefert. -

Verfährt man in gleicher Verfahrensweise, wie hier beschrieben, zur Herstellung des Phenoxazinfarbstoffes, setzt jedoch als Zinkchloridkomplexverbindung die der Beispiele 8, 11, 12 oder 72 ein, so erhält man diesen Farbstoff in gleicher Qualität und Ausbeute.

Beispiel 87

25 Teile der Zinkchloridkomplexverbindung von Beispiel 7 werden in 150 Teilen N-Methyl-pyrrolidon bei einer Temperatur von 60°C 15 Stunden lang gerührt. Die erhaltene tiefblaue Lösung wird von einer geringen Menge eines unlöslichen Rückstandes abgesaugt, der mit 50 Volumenteilen N-Methyl-pyrrolidon nachgewaschen wird. Das Filtrat wird in eine Lösung von 8 Teilen Diammoniumhydrogenphosphat in 1000 Teilen Wasser eingerührt. Der Niederschlag wird bei 50°C abgesaugt und mit 200 Teilen Wasser von 50°C nachgewaschen. Das Filtrat hieraus wird mit 5 Volumenteilen einer wäßrigen

5n-Salzsäure und sodann mit 100 Teilen Natriumchlorid versetzt. Man filtriert den ausgefällten Farbstoff ab und trocknet ihn bei 60°C. Man erhält 15 Teile des Farbstoffsalzes entsprechend der in Beispiel 83 angegebenen Formel als Chlorid. Es färbt ebenso Polyacrylnitrilfasermaterialien in farbtiefen, violettblauen Tönen mit guten Echtheiten.

Beispiel 88

11,5 Teile der Zinkchloridkomplexverbindung von Beispiel 5 werden in 200 Volumenteilen Äthanol und 10 Volumenteilen Eisessig suspendiert und auf eine Temperatur von 70°C erwärmt. Man gibt eine Lösung von 3,75 Teilen des Natriumsalzes der Äthylendiamintetraessigsäure in 20 Teilen Wasser, die mit Eisessig auf einen pH-Wert von 7 gestellt war, hinzu. Sodann versetzt man das Ganze innerhalb von etwa 5 Minuten mit 2,3 Teilen festem Natriumnitrit und rührt das Reaktionsgemisch 8 Stunden bei 70°C. Es wird die dunkelblaue Lösung des Phenoxazinfarbstoffes der Formel

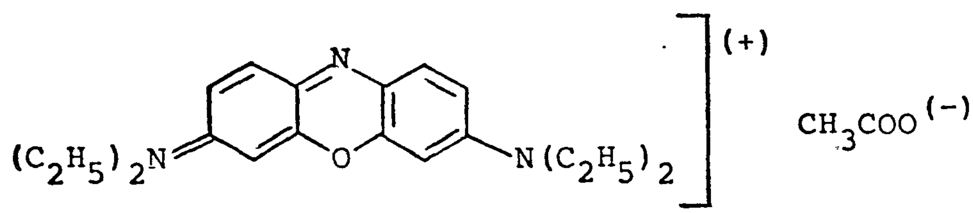

erhalten, die in üblicher Weise, wie in den vorherigen Beispielen beschrieben, aufgearbeitet werden kann.

Beispiel 89

Man suspendiert analog zum Beispiel 88 11,5 Teile der in Beispiel 5 beschriebenen Zinkchloridkomplexverbindung in 200 Volumenteilen Äthanol und 10 Volumenteilen Eisessig, gibt bei 70°C die wäßrige Lösung von 3,75 Teilen des Natriumsalzes der Äthylendiamintetraessigsäure in Wasser mit einem pH-Wert von 7 hinzu und versetzt sodann innerhalb von etwa 5 Minuten tropfenweise mit 4,8 Teilen einer wäßrigen, 40%igen Lösung von Peressigsäure. Diesen Reaktionsansatz rührt man noch 8 Stunden bei 70°C und erhält auf diese Weise eine dunkelblaue Lösung des Phenoxazinfarbstoffes, der in üblicher Weise aufgearbeitet werden kann.

Patentansprüche:

1. Zinkchloridkomplexverbindungen entsprechend der allgemeinen Formel (1)

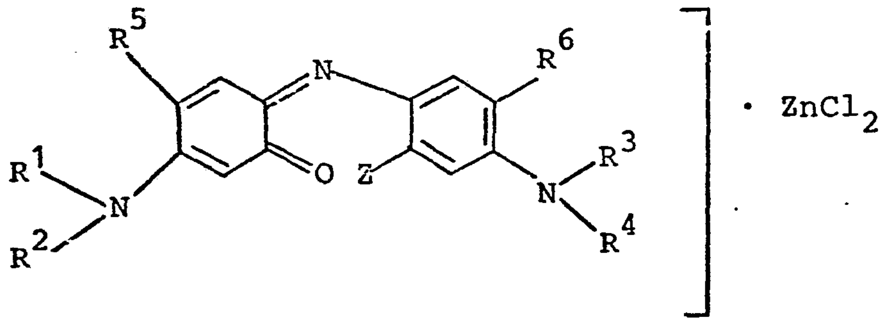

(1)

in welcher bedeuten:

$R^1$ ist ein Wasserstoffatom oder eine niedere Alkylgruppe, die substituiert sein kann;

$R^2$ ist ein Wasserstoffatom oder eine niedere Alkylgruppe, die substituiert sein kann, oder ein Phenylrest, der substituiert sein kann;

$R^3$ ist ein Wasserstoffatom oder eine niedere Alkylgruppe, die substituiert sein kann;

$R^4$ ist ein Wasserstoffatom oder eine niedere Alkylgruppe, die substituiert sein kann, oder ist ein Phenylrest, der substituiert sein kann;

$R^5$ ist ein Wasserstoffatom, ein Halogenatom, eine niedere Alkylgruppe oder eine niedere Alkoxygruppe, oder

$R^1$ und $R^5$ bilden zusammen den o-Phenylenrest;

$R^6$ ist ein Wasserstoffatom, ein Halogenatom, eine niedere Alkylgruppe oder eine niedere Alkoxygruppe;

Z ist ein Wasserstoffatom, ein Halogenatom, eine niedere Alkylgruppe, eine Cyan-, Carbonsäure-, Carbamoyl-, Sulfamoyl-, Trifluormethyl- oder niedere Carbalkoxygruppe oder eine niedere Alkoxygruppe, die durch eine Cyangruppe substituiert sein kann, eine Acyloxygruppe, eine Acylaminogruppe, eine niedere Alkylaminogruppe oder eine niedere Dialkylaminogruppe.

2. Verfahren zur Herstellung der Verbindungen von Anspruch 1, dadurch gekennzeichnet, daß man eine Nitrosoverbindung der allgemeinen Formel (2)

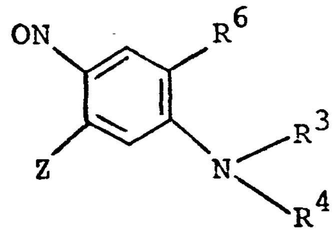

(2)

in welcher $R^3$, $R^4$, $R^6$ und Z die in Anspruch 1 genannten Bedeutungen haben, mit einem m-Aminophenol der allgemeinen Formel (3)

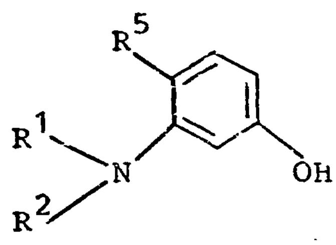

(3)

in welcher $R^1$, $R^2$ und $R^5$ die in Anspruch 1 genannten Bedeutungen haben, in Gegenwart von Zinkchlorid und in Abwesenheit einer Base umsetzt.

3. Verwendung der Verbindungen von Anspruch 1 oder der nach Anspruch 2 hergestellten Verbindungen zur Herstellung von Phenoxazinverbindungen.

4. Verfahren zur Herstellung von Phenoxazinverbindungen der allgemeinen Formel

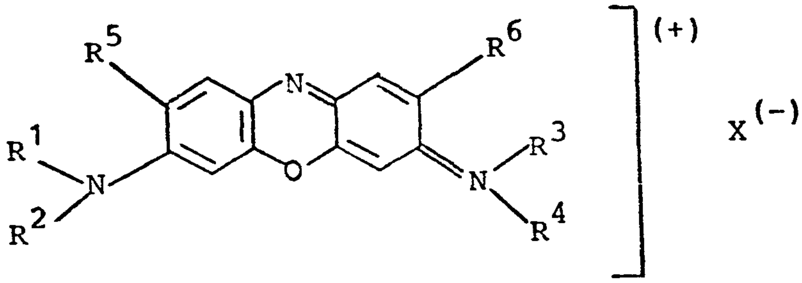

in welcher $R^1$, $R^2$, $R^3$, $R^4$, $R^5$ und $R^6$ die in Anspruch 1 genannten Bedeutungen haben und $X^{(-)}$ für ein Anion steht, dadurch gekennzeichnet, daß man eine Verbindung der in Anspruch 1 angegebenen allgemeinen Formel (1), in welcher $R^1$, $R^2$, $R^3$, $R^4$, $R^5$ und $R^6$ die in Anspruch 1 genannten Bedeutungen haben und Z für eine niedere Alkoxygruppe, die durch eine Cyangruppe substituiert sein kann, für eine Acyloxygruppe, eine Acylaminogruppe, eine niedere Alkylaminogruppe oder eine niedere Dialkylaminogruppe steht, in einem protischen oder aprotischen, polaren Lösungs- oder Verdünnungsmittel oder Mischungen derselben bei einer Temperatur zwischen etwa 5 und 120°C und gegebenenfalls in Gegenwart einer Säure oder einer Base bei einem pH-Wert zwischen 2 und 14 und/oder gegebenenfalls in Gegenwart einer Verbindung, die das komplexgebundene Zink aus der Verbindung der allgemeinen Formel (1) als schwerlösliche Zinkverbindung abscheidet oder dieses in ein lösliches Zinksalz oder Zinkat überführt, behandelt.

Europäisches Patentamt

# EUROPÄISCHER RECHERCHENBERICHT

0036555

Nummer der Anmeldung
EP 81 10 1745

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.) |
|---|---|---|---|
| A | CHEMICAL ABSTRACTS, Band 79, Nr. 1, 9. Juli 1973, Seite 432, Nr. 5096b<br>Columbus, Ohio, U.S.A.<br>I. PERINA et al.: "Oxidations with selenoxides. IV. Reaction of odihydroxy phenols with diphenyl selenoxide and diphenylselenium dichloride"<br>& BULL. SCI., CONS. ACAD. SCI. ARTS RSF YOUGOSLAVIE, SECT. A, 1973, 18(1-3),3-4<br>* Zusammenfassung *<br>---- | 1 | C 07 C 119/00<br>C 07 D 265/38<br>C 09 B 19/00 |

RECHERCHIERTE SACHGEBIETE (Int. Cl.)

C 07 C 119/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 03-07-1981 | GAUTIER |